# FASCICULE DE BREVET EUROPEEN

(11) **EP 0 850 932 B1**
(45) Date de publication et mention de la délivrance du brevet: **22.07.2009**
(21) Numéro de dépôt: 97403190.8
(22) Date de dépôt: 30.12.1997
(51) Int. Cl.: C07D 239/60, C07D 285/16, C07D 285/15, C07D 319/06, C07D 327/00, C07D 487/22, C07F 7/18, C07F 17/00, C09K 3/00, H01M 6/16, H01M 10/40, C07B 41/00, C08F 4/00, C08J 3/24, C08G 73/02, C08F 220/44, C08G 75/00

(54) **Sels d'anions hétérocycliques, et leurs utilisations comme matéreiaux à conductin ionique**
Salze von heterocyclischen Anionen und ihre Verwendungen als ionisch leitfähige Materialen
Salts of heterocyclic anions and their uses as ionic conductive materials

(30) Priorité: 30.12.1996 CA 2194127; 05.03.1997 CA 2199231
(43) Date de publication de la demande: 01.07.1998
(73) Titulaire: CENTRE NATIONAL DE LA RECHERCHE SCIENTIFIQUE (CNRS), 75016 Paris (FR); HYDRO-QUEBEC, Montréal Québec H2Z 1A4 (CA)
(72) Inventeur: Armand, Michel, Montreal, Québec H3T 1N2 (CA); Choquette, Yves, Sainte-Julie, Québec J3E 1P4 (CA); Gauthier, Michel, La Prairie, Québec J5R 1E6 (CA); Michot, Christophe, 38000 Grenoble (FR)
(74) Mandataire: Sueur, Yvette

(56) Documents cités:
- EP-A- 0 101 974
- EP-A- 0 455 300
- WO-A-88/03331
- US-A- 2 959 475
- US-A- 4 105 525
- D.V. PESTOV ET AL.: "Features of the hydrolysis of 5-(anilinomethylene) 1,3-dimethylbarbituric acid" JOURNAL OF GENERAL CHEMISTRY OF THE USSR, vol. 58, no. 8(2), août 1989, NEW-YORK, US, pages 1726-7, XP002060789
- K. SCHANK ET AL.: "Ozonolytic fragmentation of phenyliodonium beta-diketonates; a convenient synthesis of unsolvated vic-triketones" SYNTHESIS, no. 5, mai 1983, STUTTGART, DE, pages 392-5, XP002060790
- W. KANTLEHNER ET AL.: "Beiträge zur Chemie von Bis(dilakylamino)malonitrilen" LIEBIGS ANNALEN DER CHEMIE, no. 10, octobre 1990, WEINHEIM, DE, pages 965-73, XP002060791
- Francis A. Carey and Richard J. Sundberg : *dAdvanced Organic Chemistry*d, 1993, Plenum Press (third edition), New York, pages 196-206
- C. Duval et R. Duval : *dDictionnaire de la Chimie et de ses Applications*d, 1978, Technique et Documentation, troisième édition, Paris
- Paul Arnaud, Cours de Chimie Organique, Dunod, p.21-22 et 227-228*r*n
- Règles de nomenclature pour la chimie organique, 1965, règles B-1, C-61 à C-63 et D6.6 à D6.73
- POPOVYCH ET AL: "Nonaqueous solution chemistry" WILEY INTERSCIENCE , NEW YORK
- Paul Arnaud, Cours de Chimie Organique, Dunod, p.21-22 et 227-228

## Description

La présente invention a pour objet des composés ioniques dans lesquels la charge anionique est délocalisée, et leurs utilisations.

Les dérivés des anions non nucléophiles ou peu basiques présentent une importance croissante dans toutes les applications de la chimie pour stabiliser ou activer des charges cationiques diverses telles que celles des colorants, des espèces intermédiaires dans les polymérisations, ou celles qui interviennent comme intermédiaires pour des réactions variées de la chimie organique. En électrochimie, il est de plus en plus fait appel à des milieux autres que l'eau pour des applications telles que les générateurs primaires ou secondaires, les supercapacités, les systèmes de modulation de la lumière. L'introduction d'une faible conductivité ionique dans les matériaux usuels (polymères, liquides combustibles), permet de dissiper les charges électrostatiques.

On connaît principalement les dérivés des anions de coordination, de type BF4⁻, PF₆⁻, AsF₆⁻, mais ceux-ci présentent un stabilité limitée du fait de l'équilibre de dissociation libérant l'anion fluorure et l'acide de Lewis correspondant, les deux amenant des réactions parasites et présentant une toxicité non négligeable. L'anion perchlorate C10⁻ est thermiquement instable et dangereux. On connaît par ailleurs les anions dérivés des perfluoroalkylsulfonates et plus particulièrement des bis (perfluoroalkylsulfonyl)imides qui présentent des propriétés intéressantes. Mais ce type de chimie est relativement difficile à maîtriser, en particulier lors de la préparation des précurseurs de type R_{F}SO₂⁻.

On connaît par ailleurs la pyrimidinetrione (acide barbiturique) et ses dérivés obtenus en remplaçant un atome d'oxygène par un atome de soufre (acide thiobarbiturique). On connaît aussi la possibilité de former des sels avec la 2,2-diméthyl-1,3-dioxane-4,6-dione ("acide de Meldrum"). Dans les deux cas, les acides sont relativement faibles (pK_{A} de l'ordre de 5 dans l'eau, de l'ordre de 10 dans le dimethylsulfoxyde). Leurs sels ne sont ni facilement solubles ni facilement dissociables dans les solvants organiques. Dans le cas de la pyrimidinetrione, les liaisons hydrogène formées par les protons liés à l'azote renforcent cette insolubilité. Leur substitution par des radicaux alkyles diminue fortement la force de l'acide.

D.V. Pestov, et al. [Zhurnal Obshchei Khimii, vol.58, n° 8, pp. 1933-1934, Août 1988, p.1727] décrivent un sel de sodium d'un acide dérivé de l'acide barbiturique, et l'hyddrolyse de l'acide 5-(anilinométhylène)-1,3-diméthyl-barbiturique

K. Shank, et al. [Synthesis, n° 5, mai 1983, p.392-394] décrivent la fragmentation ozonolytique de Fragmentation of phényliodonium β-Dicétonates pour la synthèse de *vic*- tricé-tones, et notamment des sels de phényliodonium d'un dérivé de l'acide barbiturique.

W. Kantlehner, et al, [Liebigs Ann. Chem., 1990, 965-973] décrit des bis(dialkylamino)malononitrile, et notamment des sels d'ammonium et de sodium qui sont des dimères de l'acide N,N-dipéhnylbarbiturique.

US-2,959,475 décrit des dérivés de l'acide 1-phénylbar-biturique, ainsi que les sels correspondants, et leur utilisation pour le traitement des mauvaises herbes.

EP-0 455 300 décrit des dérivés d'acide barbiturique ayant des propriétés herbicides.

EP-0 101 974 décrit des composés du type thiadiazinone, ayant des propriétés herbicides.

US-4,105,525 décrit une cellule électrochimique pour la mesure de la teneur en ammonium d'un échantillon, dans laquelle l'électrolyte est une solution d'un sel d'ammonium de l'acide 5,5'-nitrilodibarbiturique.

WO 88/0331 décrit des trifluoroalkylsulfonylimidures et leur utilisation pour l'élaboration de matériau à conduction ionique.

Les inventeurs ont maintenant trouvé que, de manière surprenante, la solubilité et la dissociation des sels obtenus à partir des composés dérivés de la pyrimidinetrione et de ses homologues par des substitutions sur le carbone en position 5, ou sur les azotes en position 1 et 3 étaient considérablement augmentées lorsque les substituants ont un pouvoir attracteur électronique. Il en est de même pour les composés dérivés des 1,3-dioxane-4,6-diones et de leurs homologues qui portent un substituant électroattracteur sur le carbone 2 et/ou sur le carbone 5. Le choix des substituants et les nombreuses combinaisons possibles sur trois sites de substitution pour chaque famille donnent des matériaux variés dont il est possible moduler les propriétés physiques ou chimiques dans une large mesure. Ces composés ont des propriétés intéressantes pour les applications précitées et leur préparation fait appel à des matériaux plus faciles d'accès. Il est notamment possible d'obtenir des hétérocycles anioniques stables incorporant des quantités plus faibles de fluor, ou d'utiliser comme produits de départ des composés fluorés faciles d'accès. Certains composés peuvent éviter totalement le recours à des atomes de fluor.

Un composé de la présente invention comprend au moins une partie anionique associée à au moins une partie cationique M en nombre suffisant pour assurer la neutralité électronique de l'ensemble. Il est caractérisé en ce que M est un hydroxonium, un nitrosonium NO⁺, un ammonium -NH₄⁺, un cation métallique ayant la valence m, un cation organique ayant la valence m ou un cation organométallique ayant la valence m, et en ce que la partie anionique est un hétérocycle aromatique répondant à la formule 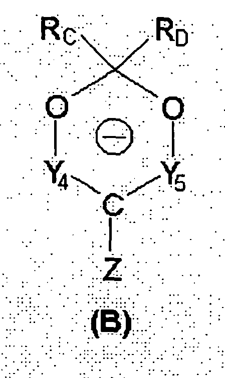 dans laquelle :
- Y₄ et Y₅ représentent chacun groupement carbonyle,
- Z représente un radical électro-attracteur choisi parmi :
   - les radicaux R_{E}Y_{E}- ou R_{E}R_{G}PO- dans lesquels Y_{E} représente un groupement carbonyle, un groupement sulfonyle ou un groupement thionyle, et R_{E} et R_{G} représentent indépendamment l'un de l'autre un halogène ou un radical organique choisi parmi :
      a) les radicaux alkyle, alkényle, oxaalkyle, oxaalkényle, azaalkyle, azaalkényle, thiaalkyle, thiaalkényle, aryle, alkylaryle, alkénylaryle, arylalkyle, arylalkényle, les radicaux alicycliques ou les radicaux aromatiques portant éventuellement au moins une chaîne latérale comprenant un hétéroatome ou comprenant éventuellement au moins un hétéroatome dans le cycle, lesdits R_{E} et R_{G} pouvant être halogénés ou perhalogénés ;
      b) les radicaux alkyles ou alkényles ayant de 1 à 12 atomes de carbone et comprenant éventuellement au moins un hétéroatome O, N ou S dans la chaîne principale ou dans une chaîne latérale, et portant un groupe hydroxy, un groupe carbonyle, un groupe amine, un groupe carboxyle, un groupe isocyanate ou un groupe thioisocyanate ;
      c) les radicaux aryle, arylalkyle, alkylaryle ou alkénylaryle, dans lesquels les noyaux aromatiques, éventuellement condensés, comprennent des hétéroatomes tels que l'azote, l'oxygène, le soufre ;
      d) les radicaux possédant un groupement iodonium, sulfonium, oxonium, ammonium, amidinium, guanidinium, pyridinium, imidazolium, triazolium, phosphonium ou carbonium, ledit groupement jouant totalement ou partiellement le rôle du cation M ;

      - -F, -Cl, -Br, -CN, -NO₂, -SCN et -N₃ ;
      - -CnF₂ₙ₊₁, -O-CnF₂ₙ₊₁, -S-CnF₂ₙ₊₁, -CH₂-CₙF₂ₙ₊₁, -OCF=CF₂ ou -SCF=CF₂, 1≤n≤8 ;
      - les radicaux ayant une valence v au moins égale à 2 et reliant v groupes ioniques 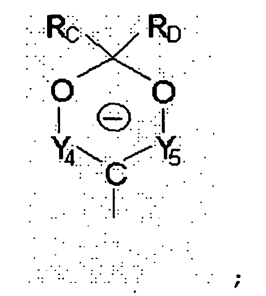
- chacun des substituants R_{C} et R_{D} représente indépendamment de l'autre un radical organique monovalent ou divalent choisi parmi :
   a. un alkyle, un alkényle, un oxa-alkyle, un oxa-alkényle, un aza-alkyle, un aza-alkényle, un thia-alkyle, un thia-alkényle, lesdits radicaux portant éventuellement au moins un groupe aryle ;
   b. un aryle portant au moins éventuellement au moins un radical alkyle, alkényle, oxa-alkyle, oxa-alkényle, aza-alkyle, aza-alkényle, thia-alkyle ou thia-alkényle ;
   c. un radical alicyclique ou un radical aromatique portant éventuellement au moins une chaîne latérale comprenant un hétéroatome ou comprenant éventuellement au moins un hétéroatome dans le cycle ;
   d. un radical tel que défini ci-dessus en a), b) et c) et portant en outre des atomes d'halogène, sous la forme halogénée ou perhalogénée
ou fait partie d'une chaîne polymère, l'un au moins des substituant R_{C} et R_{D} étant un radical perfluoré.

De préférence, le radical organique a de 1 à 20 atomes de carbone.

Dans un composé de la présente invention, le cation peut être un cation métallique choisi parmi les cations de métaux alcalins, les cations de métaux alcalino-terreux, les cations de métaux de transition, les cations de métaux trivalents, les cations de terres rares. A titre d'exemple, on peut citer Na⁺, Li⁺, K⁺, Sm³⁺, La³⁺, Ho³⁺, Sc³⁺, Al³⁺, Y³⁺, Yb³⁺, Lu³⁺, Eu³⁺.

Le cation peut également être un cation organo-métallique, notamment un métallocénium. A titre d'exemple, on peut citer les cations dérivés du ferrocène, du titanocène, du zirconocène, d'un indénocénium ou d'un arène métallocénium, les cations des métaux de transition complexés par des ligands de type phosphine possédant éventuellement une chiralité, les cations organométalliques possédant un ou plusieurs groupements alkyles ou aryles fixés d'une manière covalente à un atome ou un groupe d'atome, tels les cations méthylzinc, phénylmercure, trialkylétain ou trialkylplomb. Le cation organo-métallique peut faire partie d'une chaîne polymère.

Selon une variante de l'invention, les composés de l'invention ont un cation organique choisi dans le groupe constitué par les cations R₃O⁺ (oxonium), NR₄⁺ (ammonium), RC (NHR₂)₂⁺ (amidinium), C(NHR₂)₃⁺ (guanidinium) , C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃C⁺ (carbonium). Dans un cation donné, les radicaux R peuvent être tous identiques. Mais un cation peut aussi comporter des radicaux R différents les uns des autres. Un radical R peut être un H ou bien il est choisi parmi les radicaux suivants :
- les radicaux alkyles, alkényles, oxa-alkyles, oxa-alkényles, aza-alkyles, aza-alkényles, thia-alkyles, thia-alkényles, sila-alkyles, sila-alkényles, aryles, arylalkyles, alkyl-aryles, alkényl-aryles, dialkylamino et dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore.

Lorsqu'un cation onium porte au moins deux radicaux R différents de H, ces radicaux peuvent former ensemble un cycle aromatique ou non, englobant éventuellement le centre portant la charge cationique.

Lorsque la partie cationique d'un composé de l'invention est un cation onium, il peut se présenter soit sous la forme d'un groupe cationique indépendant qui n'est lié à la partie anionique que par la liaison ionique entre la charge positive du cation et la charge négative de la partie anionique. Dans ce cas, la partie cationique peut faire partie d'une unité récurrente d'un polymère.

Un cation onium peut également faire partie du radical Z porté par le noyau aromatique anionique. Dans ce cas, un composé de l'invention constitue un zwitterion.

Lorsque le cation d'un composé de l'invention est un cation onium, il peut être choisi de telle sorte à introduire dans le composé des substituants permettant de conférer audit composé des propriétés spécifiques. Par exemple, le cation M⁺ peut être un hétérocycle cationique à caractère aromatique, comportant au moins un atome d'azote alkylé dans le cycle. A titre d'exemple, on peut citer un imidazolium, un triazolium, un pyridinium, un 4-diméthylamino-pyridinium, lesdits cations portant éventuellement un substituant sur les atomes de carbone du cycle. Parmi ces cations, ceux qui donnent un composé ionique selon l'invention dont le point de fusion est inférieur à 150°C sont particulièrement préférés. Un tel composé possédant une température de fusion basse est particulièrement utile pour l'élaboration de matériaux à conduction protonique. Un matériau à conduction protonique particulièrement préféré comprend un composé selon l'invention dans lequel le cation est formé par addition d'un proton sur l'azote d'un imidazole ou d'un triazole, ainsi que la base azotée correspondante dans une proportion de 0,5 à 10 en rapport molaire.

Un composé de l'invention dans lequel le cation M est un groupement cationique possédant une liaison -N=N-, -N=N⁺, un groupement sulfonium, un groupement iodonium, ou un cation arène-ferrocénium substitué ou non, éventuellement incorporé dans une trame polymérique, est intéressant dans la mesure où -il est activable par une source d'énergie actinique de longueur d'onde appropriée. Comme exemples particuliers de tels composés, on peut citer ceux dans lesquels le cation est un cation diaryliodonium, un cation dialkylaryliodonium, un cation triarylsulfonium, un cation trialkylaryle sulfonium, ou un cation phénacyl-dialkyl sulfonium substitué ou non. Les cations précités peuvent faire partie d'une chaîne polymère.

Le cation M d'un composé de l'invention peut incorporer un groupement 2,2'[Azobis(2-2'-imidazolinio-2-yl)propane]²⁺ ou 2,2'-Azobis(2-amidiniopropane)²⁺. Le composé de l'invention est alors capable de libérer, sous l'action de la chaleur ou d'un rayonnement ionisant, des radicaux qui permettent d'initier des réactions de polymérisation, de réticulation ou, d'une manières générale, des réactions chimiques mettant en jeu des radicaux libres. De plus, ces composés sont aisément solubles dans les solvants organiques polymères et monomères même de faible polarité, contrairement aux dérivés des anions de type Cl⁻ habituellement associés à ce type de composés. Ils présentent par ailleurs une pression de vapeur négligeable contrairement aux autres amorceurs radicalaires de type peroxyde ou azo, ce qui est un avantage considérable pour la mise en oeuvre de polymères en films minces, la volatilité de l'amorceur ayant pour conséquence une mauvaise polymérisation ou réticulation de la surface du film.

Le choix des substituants permet d'ajuster les propriétés d'un composé ionique de l'invention.

Dans un mode de réalisation de l'invention, les substituants R_{C} et R_{D} d'autre part, peuvent être indépendamment les uns des autres,
a) un alkyle, un alkényle, un oxa-alkyle, un oxa-alkényle, un aza-alkyle, un aza-alkényle, un thia-alkyle, un thia-alkényle, lesdits radicaux portant éventuellement au moins un groupe aryle ;
b) un aryle portant au moins éventuellement au moins un radical tel que défini en a) ;
c) un radical alicyclique ou un radical aromatique portant éventuellement au moins une chaîne latérale comprenant un hétéroatome ou comprenant éventuellement au moins un hétéroatome dans le cycle ;
d) un radical tel que défini ci-dessus en a), b) et c) et portant en outre des atomes d'halogène, sous la forme halogénée ou perhalogénée.

Parmi les radicaux ci-dessus, on préfère tout particulièrement les radicaux alkyles et les radicaux alkényles ayant de 1 à 10 atomes de carbone, les radicaux alkyles ou alkényles halogénés ou perhalogénés ayant de 1 à 10 atomes de carbone, et les radicaux oxa-alkyles ou oxa-alkényles ayant de 1 à 10 atomes de carbone.

Le substituant Z peut être choisi dans le groupe constitué par -F, -Cl, -Br, -CN, -NO₂, -SCN et -N₃. Z peut également être un radical -CₙF₂ₙ₊₁, -O-CₙF_{2n+1,} -S-CnF₂ₙ₊₁, -CH₂-CₙF₂ₙ₊₁, OCF=CF₂ ou -SCF=CF₂, 1≤n≤8. En outre, Z peut être un radical qui comprend un hétérocycle dérivé de la pyridine, de la pyrazine, de la pyrimidine, de l'oxadiazole ou du thiadiazole, fluoré ou non.

Dans un autre mode de réalisation, Z est un radical R_{E}Y_{E}- ou un radical R_{E}R_{G}PO- dans lesquels Y_{E} représente un groupement carbonyle, un groupement sulfonyle ou un groupement thionyle, et R_{E} et R_{G} représentent indépendamment l'un de l'autre un halogène ou un radical organique. Les substituants comprenant un groupement sulfonyle sont particulièrement préférés.

Chacun des substituants R_{E} et R_{G} peut représenter un radical alkyle, alkényle, oxaalkyle, oxaalkényle, azaalkyle, azaalkényle, thiaalkyle, thiaalkényle, aryle, alkylaryle, alkénylaryle, arylalkyle, arylalkényle, un radical alicyclique ou un radical aromatique portant éventuellement au moins une chaîne latérale comprenant un hétéroatome ou comprenant éventuellement au moins un hétéroatome dans le cycle, lesdits R_{E} et R_{G} pouvant être halogénés ou perhalogénés.

Dans un autre mode de réalisation, R_{E} et R_{G} sont choisis indépendamment l'un de l'autre parmi les radicaux alkyles ou alkényles ayant de 1 à 12 atomes de carbone et comprenant éventuellement au moins un hétéroatome O, N ou S dans la chaîne principale ou dans une chaîne latérale, et portant un groupe hydroxy, un groupe carbonyle, un groupe amine, un groupe carboxyle.

R_{E} et R_{G} peuvent également être choisis indépendamment l'un de l'autre parmi les radicaux aryle, arylalkyle, alkylaryle ou alkénylaryle, dans lesquels les noyaux aromatiques, éventuellement condensés, comprennent des hétéroatomes tels que l'azote, l'oxygène, le soufre.

Dans un mode de réalisation particulier, l'un des groupes R_{F} ou R_{G} peut être un radical possédant un groupement iodonium, un groupement sulfonium, oxonium, ammonium, amidinium, guanidinium, pyridinium, imidazolium, imidazolinium, triazolium, phosphonium ou carbonium, ledit groupement ionique jouant totalement ou partiellement le rôle du cation M. Le composé de l'invention constitue alors un zwitterion.

Lorsque R_{E} ou R_{G} comporte au moins une insaturation éthylénique et/ou un groupe condensable et/ou un groupe dissociable par voie thermique, par voie photochimique ou par dissociation ionique, les composés de l'invention sont des composés réactifs qui peuvent être soumis à des polymérisations, des réticulations ou des condensations, éventuellement avec d'autres monomères. Ils peuvent également être utilisés pour fixer des groupes ionophores sur les polymères portant la fonction réactive.

Un substituant R_{E} ou R_{G} peut être un groupe mésomorphe ou un groupe chromophore ou un polymère conducteur électronique autodopé ou un alcoxysilane hydrolysable.

Un substituant R_{E} ou R_{G} peut comporter un groupement susceptible de piéger les radicaux libres tel que par exemple un phénol encombré ou une quinone.

Un substituant R_{E} ou R_{G} peut aussi comporter un dipôle dissociant tel que, par exemple, une fonction amide, une fonction sulfonamide ou une fonction nitrile.

Un substituant R_{E} ou R_{G} peut aussi comporter un couple rédox, par exemple un groupe disulfure, un groupe thioamide, un groupe ferrocène, un groupe phénothiazine, un groupe bis(dialkylaminoaryle), un groupe nitroxyde ou un groupe imide aromatique.

Un substituant R_{E} ou R_{G} peut comporter ou un ligand complexant ou un groupe optiquement actif.

Un substituant R_{E}-Y_{E}- représente un acide aminé, ou un polypeptide optiquement ou biologiquement actif.

Selon une autre variante, un composé selon l'invention comprend un substituant Z qui représente un radical ayant une valence v supérieure à deux, comportant lui-même au moins l'un des groupements hétérocycliques aromatiques anioniques

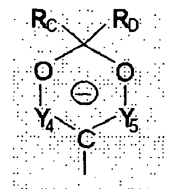

Dans ce cas, les charges négatives présentes sur la partie anionique du composé de l'invention devront être compensées par le nombre approprié de cations ou de groupes ionophores cationiques M.

Dans un mode de réalisation particulier, le radical Z multivalent est un radical bivalent comprenant au moins un groupe -SO₂-, un groupe -CO-, un groupe perfluoroalkylène ayant de 2 à 8 atomes de carbone, un groupe phénylène éventuellement substitué par des hétéroatomes, un groupement rédox -(W=W)ₙ- ou un groupement cationique -(W=W)ₙ -W⁺-, dans lesquels W représente un atome d'azote ou un groupement -C(R)-, R représentant un atome d'hydrogène ou un radical organique et 0≤n≤5. La présence du groupement cationique -(W=W)ₙ -W⁺- confère au composé de l'invention des propriétés de colorant très utiles pour les lasers. R a de préférence de 1 à 8 atomes de carbone, ou bien deux radicaux R portés par des atomes de carbone adjacents formant un cycle. Dans un autre mode de réalisation, Z est partie d'une unité récurrente d'une chaîne polymère. Le composé de l'invention présente alors des propriétés de polyélectrolyte.

R_{E} ou R_{G} peuvent également faire partie d'un radical poly(oxyalkylène) ou d'un radical polystyrène.

Un composé de la présente invention dans lequel l'anion répond à la formule générale B ci-dessus peut être préparé selon le schéma réactionnel suivant :

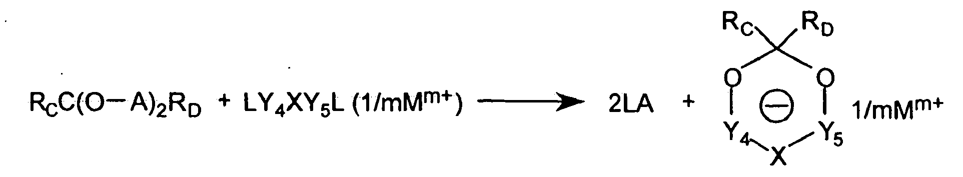

Dans tous les cas :
- L: représente un groupe partant électronégatif choisi parmi F, Cl, Br, N-imidazoyle, N-triazoyle, R_{F}-O-, R_{F}CH₂-O- et R_{F}SOₓ-, R_{F} étant un radical perfluoralkyle ;
- A: représente un cation M⁺, un groupe trialkylsilyle, un groupe trialkyle germanyle, un groupe trialkylstannyle ou un groupe tertioalkyle, dans lesquels les substituants alkyles ont de 1 à 6 atomes de carbone.

Il est avantageux dans le cas ou A = H de permettre le déplacement de la réaction dans le sens de la formation du composé de l'invention par addition d'une base tertiaire ou encombrée T susceptible de former le sel L-[HT⁺] par combinaison avec le proton.

Les bases tertiaires préférées sont en particulier choisies parmi les alkylamines, par exemple la triéthylamine, la di-isopropyléthylamine, la quinuclidine ; le 1,4 diazabicyclo[2,2,2]octane (DABCO) ; les pyridines, par exemple la pyridine, les alkylpyridines, les dialkylaminopyridines ; les imidazoles, par exemple les N-alkylimidazoles, l'imidazo[1,2-a]pyridine ; les amidines, par exemple le 1,5 diazabicyclo-[4,3,0]non-5-ène (DBN), le 1,8 diazabicyclo[5, 4, 0]undec-7-ène (DBU) ; les guanidines, par exemple la tétraméthyl guanidine, la 1,3,4,7,8-hexahydro-1-méthyl-2H-pyrimido[1,2-a]pyrimidine (HPP).

L'utilisation d'un composé R_{C}-C(O-A)₂-R_{D} lequel A est un groupement tertioalkyle est avantageuse, car un tel groupement est précurseur de proton par formation de l'alcène correspondant suivant la réaction (CH₃)₃C- ⇒ (CH₃)₂C-CH₂ + H-.

L'utilisation d'un composé R_{C}-C(O-A)₂-R_{D} lequel A est un groupe trialkylsilyle est spécialement intéressante lorsque le groupe partant est un atome de fluor, en raison de la très grande stabilité de la liaison F-Si.

Dans tous ces composés, X représente -CH- ou -C(Z)-. Les composés obtenus avec X = CH peuvent ensuite être modifiés par subsitution du proton résiduel, par exemple par action de l'anhydride trifluorométhane sulfonique.

Dans les cas où au moins un des Yᵢ est un groupement -C(=NCN)- ou un groupement -C(=C(CN)₂)-, les procédés pour obtenir ces groupements sont connus de l'homme de métier. A titre d'exemple, on peut citer la réaction d'un groupement carbonyle avec la cyanamide ou le malononitrile.

Les composés ioniques de la présente invention comprennent au moins un groupement ionophore sur lequel sont fixés des substituants qui peuvent être très variés. Compte tenu du grand choix possible pour les substituants, les composés de l'invention permettent d'induire des propriétés de conduction ionique dans la plupart des milieux organiques, liquides ou polymères possédant une polarité, même faible. Les applications sont importantes dans le domaine de l'électrochimie, en particulier du stockage de l'énergie dans des générateurs primaires ou secondaires, dans les supercapacités, dans les piles à combustibles et dans les diodes électroluminescentes. La compatibilité des composés ioniques de l'invention avec les polymères ou les liquides organiques permet d'induire des propriétés antistatiques marquées, même lorsque la teneur en composé ionique est extrêmement faible. Les composés de l'invention qui sont des polymères, de même que des composés polymères obtenus à partir de composés de l'invention ayant la propriété de se polymériser ou de se copolymériser, présentent les propriétés énumérées ci-dessus avec l'avantage d'avoir d'une charge anionique immobile. C'est pourquoi un autre objet de la présente invention est constitué par un matériau à conduction ionique constitué par un composé ionique de la présente invention en solution dans un solvant.

Dans un mode de réalisation, le composé ionique utilisé pour l'élaboration d'un matériau à conduction ionique est choisi parmi les composés dont le cation est l'ammonium, ou un cation dérivé d'un métal, en particulier le lithium ou le potassium, le zinc, le calcium, les métaux des terres rares, ou un cation organique, tel qu'un ammonium substitué, un imidazolium, un triazolium, un pyridinium, un 4-diméthylamino-pyridinium, lesdits cations portant éventuellement un substituant sur les atomes de carbone du cycle. Le matériau à conduction ionique ainsi obtenu présente une conductivité et une solubilité dans les solvants élevées, du fait des interactions faibles entre la charge positive et la charge négative. Son domaine de stabilité électrochimique est étendu, et il est stable dans des milieux aussi bien réducteurs qu'oxydants. De plus, les composés qui ont un cation organique et un point de fusion inférieur à 150°C, en particulier les composés de imidazolium, de triazolium, de pyridinium, de 4-diméthylamino-pyridinium présentent une conductivité élevée intrinsèque, même en l'absence de solvant, lorsqu'ils sont en phase fondue.

Les propriétés du matériau à conduction ionique peuvent également être adaptées par le choix du substituant R_{C}, R_{D}, R_{E} et R_{G}.

Le choix pour l'un au moins des substituants R_{C}, R_{D,} R_{E} ou R_{G} d'un groupe alkyle, d'un groupe aryle, d'un groupe alkylaryle ou d'un groupe arylalkyle, permet d'induire dans le matériau à conduction ionique des propriétés de type mésogène, en particulier les groupements alkyles de 6 à 20 atomes de carbones, les groupements aryle-alkyle, en particulier ceux contenant l'entité biphényle qui forment des phases de type cristal liquide. Des propriétés de conduction dans des phases de type cristal liquide, nématique, cholestérique ou discotique, sont intéressantes pour les applications relatives à l'affichages optique ou pour réduire la mobilité des anions dans les électrolytes, en particulier dans les électrolytes polymères, sans affecter la mobilité des cations. Cette particularité est importante pour les applications dans les générateurs électrochimiques, en particulier ceux mettant en jeu les cations lithium.

Lorsque le substituant Z est un groupe mésomorphe ou un groupe comprenant au moins une insaturation éthylénique et/ou un groupe condensable et/ou un groupe dissociable par voie thermique, par voie photochimique ou par dissociation ionique, le matériau à conduction ionique forme aisément des polymères ou copolymères qui sont des polyélectrolytes, soit intrinsèques quand le polymère porte des groupements solvatants, soit par addition d'un solvant polaire de type liquide ou polymère, ou par mélange avec un tel solvant. Ces produits ont une conductivité uniquement due au cations, ce qui constitue une propriété très utile dans les applications de type générateur électrochmique. En faible fraction molaire dans un copolymère, ils induisent des propriétés antistatiques stables et peu dépendantes de l'humidité et favorisent la fixation de colorants cationiques, cette propriété étant utile pour les fibres textiles et les lasers à colorants.

La présence d'un substituant Z qui est un polymère conducteur électronique autodopé, améliore la stabilité du matériau à conduction ionique par rapport aux agents extérieurs. La conductivité est stable dans le temps même à des températures élévées. En contact avec les métaux, ces matériaux donnent des résistances d'interface très faibles et protègent en particulier les métaux ferreux ou l'aluminium de la corrosion.

Lorsque Z est un alcoxysilane hydrolysable, le matériau à conduction ionique peut former des des polymères stables par simple mécanisme d'hydrolyse-condensation en présence d'eau, permettant ainsi de traiter les surfaces d'oxydes, de silice, de silicates, en particulier le verre, pour induire des propriétés de conduction de surface, des propriétés antistatiques, ou pour favoriser l'adhésion de polymères polaires.

Lorsque Z est un groupe comprenant un piège à radicaux libres tel qu'un phénol encombré, ou une quinone, le matériau à conduction ionique présente les avantages et propriétés suivantes : il agit comme antioxydant ne présentant pas de volatilité et compatible avec les monomères et polymères polaires, auquel il confère de surcroît des propriétés antistatiques.

Lorsque Z comprend un dipôle dissociant tel qu'une amide, une sulfonamide ou un nitrile, le matériau à conduction ionique a une conductivité améliorée dans des milieux de faible et moyenne polarité, en particulier dans les polymères solvatants, ce qui permet de minimiser, voire de supprimer l'addition de solvants ou de plastifiants volatils.

La présence d'un substituant Z qui contient un couple rédox tel qu'un disulfure, une thioamide, un ferrocène, une phéno-thiazine, un groupe bis(dialkylaminoaryle), un nitroxyde, un imide aromatique, permet d'induire dans le matériau à conduction ionique des propriétés de navette rédox utiles comme élément de protection et d'égalisation de charge des générateurs électrochimiques, dans les systèmes photoélectrochimiques, en particulier de conversion de la lumière en électricité, dans les systèmes de modulation de la lumière de type électrochrome.

La présence d'un substituant Z qui est un ligand complexant dans un matériau à conduction ionique permet de chélater les cations métalliques, en particulier ceux qui possèdent un charge élevée (2, 3 et 4), sous forme de complexe soluble dans les milieux organiques, y compris dans les milieux aprotiques, et permet le transport de ces cations en particulier sous forme de complexe anionique, dans les polymères solvatants. Les cations métalliques de charge élevée sont en effet immobiles dans les polymères solvatants. Ce type de complexant donne avec certains cations des métaux de transition (Fe, Co...) ou de certaines terres rares (Ce, Eu...) des couples rédox particulièrement stables.

Les matériaux à conduction ionique contenant un composé de l'invention dans l'un au moins des substituants R_{C}, R_{D}, R_{E} ou R_{G} est un substituant alkyle ou alkényle qui contient au moins un hétéroatome choisi parmi O, N et S ont un pouvoir complexant, et plastifiant, en particulier dans les polymères polaires et tout specialement les polyéthers. Les hétéroatomes N et S sont sélectivement complexants pour les cations des métaux de transition, Zn et Pb.

Lorsqu'un substituant R_{E} ou R_{G} alkyle ou alkényle porte en outre un groupe hydroxy, un groupe carbonyle, un groupe amine, un groupe carboxyle, un groupe isocyanate ou un groupe thioisocyanate, le composé ionique de l'invention peut donner par polycondensation un polymère ou un copolymère et le matériau à conduction ionique qui contient un tel polymère ou copolymère présente des propriétes de polyélectrolyte.

La présence, dans le matériau à conduction ionique de l'invention, d'un composé dans lequel l'un au moins des substituants R_{E} ou R_{G} est choisi parmi les radicaux aryle, arylalkyle, alkylaryle ou alkénylaryle, dans lesquels les chaînes latérales et/ou les noyaux aromatiques comprennent des hétéroatomes tels que l'azote, l'oxygène, le soufre, améliore la dissociation et augmente la possibilité de former des complexes suivant la position de l'hétéroatome (pyridine) ou de donner par oxydation duplicative des polymères ou copolymères conjugués (pyrrole, thiophène).

Lorsque le matériau à conduction ionique contient un composé de l'invention dans lequel Z représente une unité récurrente d'une chaîne polymère, le matériau constitue un polyélectrolyte.

Un composé de l'invention dans lequel le substituant Z est choisi dans le groupe constitué par -OCₙF₂ₙ₊₁. -OC₂F₄H, -SCₙF₂ₙ₊₁, -SC₂F₄H, -OCF=CF₂ ou -SCF=CF₂, n étant un nombre entier de 1 à 8, est un précurseur de monomères et polymères stables, en particulier vis-à-vis de l'oxygène même à des températures supérieures à 80°C lorsqu'il s'agit des polymères. Un matériau à conduction ionique qui contient un tel composé est donc particulièrement approprié comme électrolyte d'une pile à combustible.

Un matériau à conduction ionique de la présente invention comprend un composé ionique de l'invention en solution dans un solvant.

Le solvant peut être un solvant liquide aprotique, un polymère polaire ou un de leurs mélanges.

Le solvant liquide aprotique est choisi par exemple parmi les éthers linéaires et les éthers cycliques, les esters, les nitriles, les dérivés nitrés, les amides, les sulfones, les sulfolanes, les alkylsulfamides et les hydrocarbures partiellement halogénés. Les solvants particulièrement préférés sont le diéthyléther, le diméthoxyéthane, le glyme, le tétrahydrofurane, le dioxane, le diméthyltétra-hydrofurane, le formiate de méthyle ou d'éthyle, le carbonate de propylène ou d'éthylène, les carbonates d'alkyles (notamment le carbonate de diméthyle, le carbonate de diéthyle et le carbonate de méthylpropyle), les butyrolactones, l'acétonitrile, le benzonitrile, le nitrométhane, le nitrobenzène, la diméthylformamide, la diéthylformamide, la N-méthyl-pyrrolidone, la diméthylsulfone, la tétraméthylène sulfone et les tétraalkylsulfonamides ayant de 5 à 10 atomes de carbone.

Le polymère polaire peut être choisi parmi les polymères solvatants, réticulés ou non, portant ou non des groupes ioniques greffés. Un polymère solvatant est un polymère qui comporte des unités solvatantes contenant au moins un hétéroatome choisi parmi le soufre, l'oxygène, l'azote et le fluor. A titre d'exemple de polymères solvatants, on peut citer les polyéthers de structure linéaire, peigne ou à blocs, formant ou non un réseau, à base de poly(oxyde d'éthylène), ou les copolymères contenant le motif oxyde d'éthylène ou oxyde de propylène ou allylglycidyléther, les polyphosphazènes, les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox. Bien entendu, la liste ci-dessus n'est pas limitative, et tous les polymères présentant des propriétés solvatantes peuvent être utilisés.

Un matériau à conduction ionique de la présente invention peut comprendre simultanément un solvant liquide aprotique choisi parmi les solvants liquides aprotiques cités ci-dessus et un solvant polymère polaire comprenant des unités contenant au moins un hétéroatome choisi parmi le soufre, l'azote, l'oxygène et le fluor. Il peut comprendre de 2 à 98% de solvant liquide. A titre d'exemple d'un tel polymère polaire, on peut citer les polymères qui contiennent principalement des unités dérivées de l'acrylonitrile, du fluorure de vinylidène, de la N-vinylpyrrolidone ou du méthacrylate de méthyle. La proportion de liquide aprotique dans le solvant peut varier de 2% (correspondant à un solvant plastifié) à 98% (correspondant à une solvant gélifié).

Un matériau à conduction ionique de la présente invention peut contenir en outre un sel utilisé classiquement dans l'art antérieur pour l'élaboration d'un matériau à conduction ionique. Parmi les sels utilisables en mélange avec un composé ionique selon l'invention, on préfère tout particulièrement un sel choisi parmi les perfluoroalcanesulfonates, les bis(perfluoroalkylsulfonyl) imidures, les bis(perfluoroalkylsulfonyl) méthanes et les tris(perfluoroalkylsulfonyl)-méthanes.

Bien entendu, un matériau à conduction ionique de l'invention peut contenir en outre les additifs utilisés de manière classique dans ce type de matériau, et notamment des charges minérales ou organiques sous forme de poudre ou de fibres.

Un matériau à conduction ionique de l'invention peut être utilisé comme électrolyte dans un générateur électrochimique. La présente invention a ainsi pour autre objet un générateur électrochimique comprenant une électrode négative et une électrode positive séparées par un électrolyte, caractérisé en ce que l'électrolyte est un matériau à conduction ionique tel que défini ci-dessus. Selon un mode de réalisation particulier, un tel générateur comprend une électrode négative constituée par du lithium métallique, ou par l'un de ses alliages, éventuellement sous forme de dispersion nanométrique dans de l'oxyde de lithium, ou par un nitrure double de lithium et d'un métal de transition, ou par un oxyde à bas potentiel ayant pour formule générale Li_{1+y+x/3}Ti_{2-x/3}O₄ (0 ≤ x ≤ 1, 0 ≤ y ≤ 1), ou par le carbone et les produit carbonés issus de la pyrolyse de matières organiques. Selon un autre mode de réalisation, le générateur comprend une électrode positive choisie parmi les oxydes de vanadium VOₓ (2 ≤ x ≤ 2,5), LiV₃O₈, LiyNi₁₋ₓCOₓO₂, (0 ≤ x ≤ 1 ; 0 ≤ y ≤ 1), les spinelles de manganèse Li_{y}Mn₁₋ₓMₓO₂ (M = Cr, Al, V, Ni, 0 ≤ x ≤ 0,5 ; 0 ≤ y ≤ 2), les polydisulfures organiques, FeS, FeS₂, le sulfate de fer Fe₂(SO₄)₃, les phosphates et phosphosilicates de fer et de lithium de structure olivine, ou leurs produits de substitution du fer par le manganèse, utilisés seuls ou en mélanges. Le collecteur de l'électrode positive est de préférence en aluminium.

Un matériau à conduction ionique de la présente invention peut également être utilisé dans une supercapacité. Un autre objet de la présente invention est par conséquent une supercapacité utilisant au moins une électrode de carbone à haute surface spécifique, ou une électrode contenant un polymère rédox, dans laquelle l'électrolyte est un matériau à conduction ionique tel que défini ci-dessus.

Un matériau à conduction ionique de la présente invention peut également être utilisé pour le dopage p ou n d'un polymère à conduction électronique et cette utilisation constitue un autre objet de la présente invention.

En outre, un matériau à conduction ionique de la présente invention peut être utilisé comme électrolyte dans un dispositif électrochrome. Un dispositif électrochrome dans lequel l'électrolyte est un matériau à conduction ionique selon l'invention est un autre objet de la présente invention.

Il a été observé que la forte dissociation des espèces ioniques des composés de l'invention se traduisait par une stabilisation des carbocations, en particulier ceux dans lesquels il existe une conjugaison avec l'oxygène ou l'azote et, d'une manière surprenante, par une forte activité de la forme protonée des composés l'invention sur certains monomères. La présente invention a donc également pour objet l'utilisation des composés ioniques comme photoinitiateurs sources d'acides de Bronsted catalyseurs de polymérisation ou de réticulation de monomères ou de prépolymères capables de réagir par voie cationique, ou comme catalyseurs pour la modification de polymères.

Le procédé de polymérisation ou de réticulation de monomères ou de prépolymères capables de réagir par voie cationique est caractérisé en ce que l'on utilise un composé de l'invention comme photoinitiateur source d'acide catalysant la réaction de polymérisation. Les composés selon l'invention dans lesquels le cation est un groupement possédant une liaison -N=N⁺, -N=N- un groupement sulfonium, un groupement iodonium, ou un cation arène-ferrocénium substitué ou non, éventuellement incorporé dans une trame polymérique, sont particulièrement préférés.

Le choix des divers substituants de l'hétérocycle anionique est effectué de manière à augmenter la solubilité dudit composé dans les solvants utilisés pour la réaction des monomères ou des prépolymères, et en fonction des propriétés souhaitées pour le polymère final. Par exemple, le choix de radicaux alkyles non substitués donne une solubilité dans les milieux peu polaires. Le choix de radicaux comprenant un groupe oxa ou une sulfone donnera une solubilité dans les milieux polaires. Les radicaux incluant un groupement sulfoxyde, un groupement sulfone, un groupement oxyde de phosphine, un groupement phosphonate, obtenus respectivement par addition d'oxygène sur les atomes de soufre ou de phosphore, peuvent conférer au polymère obtenu des propriétés améliorées en ce qui concerne l'adhésion, la brillance, la résistance à l'oxydation ou aux UV. Les monomères et les prépolymères qui peuvent être polymérisés ou réticulés à l'aide des photoinitiateurs de la présente invention sont ceux qui peuvent subir une polymérisation cationique.

Parmi les monomères, on peut citer les monomères qui comportent une fonction éther cyclique, une fonction thioéther cyclique ou une fonction amine cyclique les composés vinyliques (plus particulièrement les éthers vinyliques), les oxazolines, les lactones et les lactames.

Parmi les monomères du type éther ou thioéther cyclique, on peut citer l'oxyde d'éthylène, l'oxyde de propylène, l'oxétane, l'épichlorhydrine, le tétrahydrofurane, l'oxyde de styrène, l'oxyde de cyclohexène, l'oxyde de vinylcyclohexène, le glycidol, l'oxyde de butylène, l'oxyde d'octylène, les éthers et les esters de glycidyle (par exemple le méthacrylate ou l'acrylate de glycidyle, le phényl glycidyl éther, le diglycidyléther de bisphénol A ou ses dérivés fluorés), les acétals cycliques ayant de 4 à 15 atomes de carbone (par exemple le dioxolane, le 1,3-dioxane, le 1,3-dioxépane) et les spiro-bicyclo dioxolanes.

Parmi les composés vinyliques, les éthers vinyliques constituent une famille très importante de monomères sensibles en polymérisation cationique. A titre d'exemple, on peut citer l'éthyl vinyl éther, le propyl vinyl éther, l'iso-butyl vinyl éther, l'octadécyl vinyl éther, l'éthylèneglycol monovinyl éther, le diéthylèneglycol divinyl éther, le butanediol monovinyl éther, le butanediol divinyl éther, l'hexanediol divinyl éther, l'éthylèneglycol butyl vinyl éther, le triéthylèneglycol méthyl vinyl éther, le cyclohexanediméthano monovinyl éther, le cyclohexanediméthanol divinyl éther, le 2-éthylhexyl vinyl éther, le poly-THF-divinyl éther ayant une masse comprise entre 150 et 5000, le diéthylèneglycol monovinyl éther, le triméthylolpropane trivinyl éther, l'aminopropyl vinyl éther, le 2-diéthylaminoéthyl vinyl éther.

Comme autres composés vinyliques, on peut citer à titre d'exemple les 1,1-dialkyléthylènes (par exemple l'isobutène), les monomères aromatiques vinyliques (par exemple le styrène, les α-alkylstyrènes, notamment l'α-méthylstyrène, le 4-vinylanisole, l'acénaphtène), les composés N-vinyliques (par exemple la N-vinylpyrolidone ou les N-vinyl sulfonamides).

Parmi les prépolymères, on peut citer les composés dans lesquels des groupements époxy sont portés par une chaîne aliphatique, une chaîne aromatique, ou une chaîne hétérocyclique, par exemple les éthers glycidiques du bisphénol A éthoxylés par 3 à 15 unités d'oxyde d'éthylène, les siloxanes possédant des groupements latéraux du type époxycyclohexène-éthyle obtenus par hydrosilylation des copolymères de di alkyl, d'alkylaryle ou de diaryl siloxane avec le méthyl hydrogénosiloxane en présence d'oxyde de vinylcyclohexène, les produits de condensation du type sol-gel obtenus à partir du triéthoxy ou du triméthoxy silapropylcyclohexène oxyde, les uréthanes incorporant les produits de réaction du butanediol monovinyléther et d'un alcool de fonctionnalité supérieure ou égale à 2 sur un di ou un tri isocyanate aliphatique ou aromatique.

Le procédé de polymérisation selon l'invention consiste à mélanger au moins un monomère ou prépolymère capable de polymériser par voie cationique et au moins un composé ionique de l'invention, et à soumettre le mélange obtenu à un rayonnement actinique ou un rayonnement β. De préférence, le mélange réactionnel est soumis au rayonnement après avoir été mis sous forme d'une couche mince ayant une épaisseur inférieure à 5 mm, de préférence sous forme d'un film mince ayant une épaisseur inférieure ou égale à 500 µm. La durée de la réaction dépend de l'épaisseur de l'échantillon et de la puissance de la source à la longueur d'onde λ active. Elle est définie par la vitesse de défilement devant la source, qui est comprise entre 300 m/min et 1 cm/min. Des couches de matériau final ayant une épaisseur supérieure à 5 mm peuvent être obtenues en répétant plusieurs fois l'opération consistant à épandre une couche et à la traiter par le rayonnement.

Généralement, la quantité de photoinitiateur utilisé est comprise entre 0,01 et 15 % en poids par rapport au poids de monomère ou de prépolymère, de préférence entre 0,1 et 5 % en poids.

Un composé ionique de la présente invention peut être utilisé comme photoinitiateur en l'absence de solvant, notamment lorsque l'on souhaite polymériser des monomères liquides dans lesquels le composé ionique utilisé comme photoinitiateur est soluble ou aisément dispersable. Cette forme d'utilisation est particulièrement intéressante, car elle permet de supprimer les problèmes liés aux solvants (toxicité, inflammabilité).

Un composé ionique de la présente invention peut également être utilisé en tant que photoinitiateur sous forme d'une solution homogène dans un solvant inerte vis-à-vis de la polymérisation, prête à l'emploi et aisément dispersable, en particulier dans le cas où le milieu à polymériser ou à réticuler présente une viscosité élevée.

Comme exemple de solvant inerte, on peut citer les solvants volatils, tels que l'acétone, la méthyl-éthyl cétone et l'acétonitrile. Ces solvants serviront simplement à diluer les produits à polymériser ou à réticuler (pour les rendre moins visqueux, surtout lorsqu'il s'agit d'un prépolymère). Ils seront éliminés après la polymérisation ou la réticulation par séchage. On peut également citer les solvants non volatils. Un solvant non volatil sert également à diluer les produits que l'on veut polymériser ou réticuler, et à dissoudre le composé ionique de l'invention utilisé comme photoinitiateur, mais il restera dans le matériau formé et il agit ainsi comme plastifiant. A titre d'exemple, on peut citer le carbonate de propylène, la γ-butyrolactone, les éther-esters des mono-, di-, tri- éthylène ou propylène glycols, les éther-alcools des mono-, di-, tri- éthylène ou propylène glycols, les plastifiants tels que les esters de l'acide phtalique ou de l'acide citrique.

Dans un autre mode de mise en oeuvre de l'invention, on utilise comme solvant ou diluant un composé réactif vis-à-vis de la polymérisation, qui est un composé de faible masse moléculaire et de faible viscosité qui va jouer à la fois le rôle de monomère polymérisable et le rôle de solvant ou de diluant pour des monomères plus visqueux ou des prépolymères utilisés conjointement. Après la réaction, ces monomères ayant servi de solvant font partie du réseau macromoléculaire finalement obtenu, leur intégration étant plus grande lorsqu'il s'agit de monomères bi-fonctionnels. Le matériau obtenu après irradiation ne contient plus de produits ayant un faible poids moléculaire et une tension de vapeur appréciable, ou susceptibles de contaminer les objets avec lesquels le polymère est en contact. A titre d'exemple, un solvant réactif peut être choisi parmi les mono et di éthers vinyliques des mono-, di-, tri-, tétra- éthylène et propylène glycols, la N-méthylpyrolidone, le 2-propényléther du carbonate de propylène commercialisé par exemple sous la dénomination PEPC par la société ISP, New Jersey, Etats-Unis.

Pour irradier le mélange réactionnel, le rayonnement peut être choisi parmi le rayonnement ultraviolet, le rayonnement visible, les rayons X, les rayons γ et le rayonnement β. Lorsque l'on utilise la lumière ultraviolette comme rayonnement actinique, il peut être avantageux d'ajouter aux photoinitiateurs de l'invention des photosensibilisateurs destinés à permettre une photolyse efficace avec les longueurs d'ondes moins énergétiques que celles correspondant au maximum d'absorption du photoinitiateur, telles que celles émises par les dispositifs industriels, (λ ≈ 300 nm pour les lampes à vapeur de mercure en particulier). De tels additifs sont connus, et à titre d'exemples non limitatifs, on peut citer l'anthracène, le diphényl-9,10-anthracène, le pérylène, la phénothiazine, le tétracène, la xanthone, la thioxanthone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles permettant entre autre de changer la longueur d'onde d'absorption. L'isopropylthioxantone est un exemple de photosensibilisateur préféré lorsque l'on utilise un sel d'iodonium selon l'invention comme photoinitiateur.

Parmi les différents types de rayonnement mentionnés, le rayonnement ultraviolet est particulière préféré. D'une part, il est plus commode d'emploi que les autres rayonnements mentionnés. D'autre part, les photoinitiateurs sont en général directement sensibles aux rayons UV et les photo-sensibilisateurs sont d'autant plus efficaces que la différence d'énergie (δλ) est plus faible.

Les composés ioniques de l'invention peuvent aussi être mis en oeuvre en association avec des amorceurs de type radicalaire générés thermiquement ou par action d'une radiation actinique. Il est ainsi possible de polymériser ou de réticuler des mélanges de monomères ou de prépolymères contenant des fonctions dont les modes de polymérisation sont différents, par exemple des monomères ou des prépolymères polymérisant par voie radicalaire et des monomères ou des prépolymères polymérisant par voie cationique. Cette possibilité est particulièrement avantageuse pour créer des réseaux interpénétrés ayant des propriétés physiques différentes de celles qui seraient obtenues par simple mélange des polymères issus des monomères correspondants. Les éthers vinyliques ne sont pas ou sont peu actifs par amorçage radicalaire. Il est donc possible, dans un mélange réactionnel contenant un photoinitiateur selon l'invention, un amorceur radicalaire, au moins un monomère du type éther vinylique et au moins un monomère comprenant des doubles liaisons non activées telles que celles des groupes allyliques, d'effectuer une polymérisation séparée de chaque type de monomère. Il est par contre connu que les monomères déficients en électrons, tels que les esters ou les amides de l'acide fumarique, de l'acide maléique, de l'acide acrylique ou méthacrylique, de l'acide itaconique, de l'acrylonitrile, du méthacrylonitrile, la maléimide et ses dérivés, forment en présence d'éthers vinyliques riches en électrons, des complexes de transfert de charge donnant des polymères alternés 1:1 par amorçage radicalaire. Un excès initial de monomères vinyliques par rapport à cette stoechiométrie permet de préserver des fonctions polymérisables par initiation cationique pure. Le déclenchement de l'activité d'un mélange d'amorceur radicalaire et d'amorceur cationique selon l'invention peut être fait simultanément pour les deux réactifs dans le cas par exemple d'insolation par un rayonnement actinique d'une longueur d'onde pour laquelle les photoinitiateurs de l'invention et les amorceurs radicalaires choisis sont actifs, par exemple à λ = 250 nm. A titre d'exemple, on peut citer comme amorceurs les produits commerciaux suivants : Irgacure 184^{®}, Irgacure 651^{®}, Irgacure 261^{®} Quantacure DMB^{®}, Quantacure ITX^{®}.

Il peut aussi être avantageux d'utiliser les deux modes de polymérisation d'une manière séquentielle, pour former dans un premier temps des prépolymères dont la mise en forme est aisée et dont le durcissement, l'adhésion, la solubilité ainsi que le degré de réticulation peuvent être modifiés par le déclenchement de l'activité de l'amorceur cationique. Par exemple, un mélange d'un amorceur radicalaire thermodissociable et d'un photoinitiateur cationique selon l'invention permet de réaliser des polymérisations ou des réticulations séquentielles, d'abord sous l'action de la chaleur, puis sous l'action d'un rayonnement actinique. D'une manière similaire, si l'on choisit un amorceur radicalaire et un photoinitiateur cationique selon l'invention, le premier étant photosensible à des longueurs d'ondes plus longues que celle déclenchant le photoinitiateur selon l'invention, on obtient une réticulation en deux étapes contrôlables. Des amorceurs radicalaires peuvent être par exemple Irgacure^{®} 651 permettant d'amorcer des polymérisations radicalaires à des longueurs d'onde de 365 nm.

L'invention a également pour objet l'utilisation des composés ioniques de l'invention pour les réactions d'amplification chimique de photoresists pour la microlithographie. Lors d'une telle utilisation, un film d'un matériau comprenant un polymère et un composé ionique de l'invention est soumis à une irradiation. L'irradiation provoque la formation de l'acide par remplacement du cation M par un proton, qui catalyse la décomposition ou la transformation du polymère. Après décomposition ou transformation du polymère sur les parties du film qui ont été irradiées, les monomères formés ou le polymère transformé sont éliminés et il reste une image des parties non exposées. Pour cette application particulière, il est avantageux d'utiliser un composé de l'invention qui se présente sous la forme d'un polymère constitué essentiellement d'unités récurrentes styrényles portant un hétérocycle aromatique anionique comme substituant. Ces composés permettent d'obtenir après photolyse des produits qui ne sont pas volatils, et donc pas odorants lorsqu'il s'agit de sulfures. Parmi les polymères qui peuvent ainsi être modifiés en présence d'un composé de l'invention, on peut citer notamment les polymères contenant des motifs ester ou des motifs aryléther de tertioalkyle, par exemple les poly(phtalaldéhydes), les polymères de bisphénol A et d'un diacide, le polytertiobutoxycarbonyl oxystyrène, le polytertiobutoxy-α-méthyl styrène, le polyditertiobutyl-fumarate-co-allyltriméthylsilane et les polyacrylates d'un alcool tertiaire, en particulier le polyacrylate de tertiobutyle. D'autres polymères sont décrits dans J.V. Crivello et al, Chemistry of Materials 8, 376-381, (1996).

Les composés ioniques de la présente invention, qui présentent une grande stabilité thermique, offrent de nombreux avantages par rapport aux sels connus de l'art antérieur. Ils ont notamment des vitesses d'amorçage et de propagation comparables ou supérieures à celles obtenues à l'aide des anions de coordination de type PF₆⁻, AsF₆⁻ et surtout SbF₆⁻.

Dans les composés de la présente invention, les paires d'ions présentent une très forte dissociation, ce qui permet l'expression des propriétés catalytiques intrinsèques du cation M^{m+}, dont les orbitales actives sont facilement exposées aux substrats de la réaction, ceci dans des milieux variés. La plupart des réactions importantes de la chimie organique peuvent être ainsi effectuées dans des conditions peu contraignantes, avec d'excellents rendements et la facilité de séparer le catalyseur du milieu réactionnel. La mise en évidence d'induction asymétrique par l'utilisation d'un composé ionique selon l'invention qui portent un groupement chiral est particulièrement importante de part sa généralité et sa facilité de mise en oeuvre. La présente invention a par conséquent comme autre objet l'utilisation des composés de l'invention comme catalyseurs dans les réactions de Friedel-Crafts, les réactions de Diels et Alder, les réactions d'aldolisation, les additions de Michael, les réactions d'allylation, les réactions de couplage pinacolique, les réactions de glycosilation, les réactions d'ouvertures de cycles des oxétanes, les réactions de méthathèse des alcènes, les polymérisations de type Ziegler-Natta, les polymérisations du type méthathèse par ouverture de cycle et les polymérisations du type méthathèse de diènes acycliques. Les composés ioniques de l'invention préférés pour une utilisation en tant que catalyseur pour les réactions ci-dessus sont ceux dans lesquels le cation est choisi parmi le lithium, le magnésium, le cuivre, le zinc, l'étain, les métaux trivalents, y compris les terres rares, les platinoïdes, et leurs couples organométalliques, en particulier les métallocènes.

Les composés de l'invention peuvent également être utilisés comme solvant pour effectuer des réactions chimiques, photochimiques, électrochimiques, photoélectrochimiques. Pour cette utilisation particulière, on préfère les composés ioniques dans lesquels le cation est un imidazolium, un triazolium, un pyridinium ou un 4-diméthylamino-pyridinium, ledit cation portant éventuellement un substituant sur les atomes de carbone du cycle. Les composés étant utilisés sous leur forme liquide, on préfère tout spécialement ceux qui ont un point de fusion inférieur à 150°C, plus particulièrement inférieur à 100°C.

Les inventeurs ont également trouvé que la charge anionique portée par le groupement anionique exerçait un effet stabilisant sur les conducteurs électroniques de type polymères conjugués, et que l'utilisation d'un composé dans lequel l'un des substituants comprenait une longue chaîne alkyle permettait, de rendre ces polymères solubles dans les solvants organiques usuels même à l'état dopé. Le greffage de ces charges sur le polymère lui-même donne des polymères dont la charge globale est cationique, solubles dans les solvants organiques et présentant, outre leur stabilité, des propriétés d'anticorrosion vis-à-vis des métaux, l'aluminium et les métaux ferreux. La présente invention a aussi pour objet des matériaux à conduction électronique comprenant un composé ionique de la présente invention dans lequel la partie cationique est un polycation constitué par un polymère conjugué dopé "p". Les composés ioniques préférés pour cette application sont ceux dans lesquels l'un des substituants R_{A}, R_{B}, R_{C}, R_{D} ou Z contient au moins une chaîne alkyle ayant de 6 à 20 atomes de carbone.

Les colorants de type cationique (cyanines) sont utilisés de plus en plus fréquemment comme sensibilisateurs de films photographiques, pour le stockage optique d'information (disques optiques accessibles en écriture), pour les lasers. La tendance de ces molécules conjuguées à s'empiler lorsqu'elles sont en phases solides limite leur utilisation, par suite des variations des propriétés optiques par rapport à la molécule isolée. L'utilisation de composés ioniques de l'invention pour la fabrication de colorants cationiques dont les contre ions, éventuellement fixés à cette même molécule, correspondent aux fonctionnalités de l'invention, permet de réduire les phénomènes d'agrégation, y compris dans des matrices solides polymères et de stabiliser ces colorants. La présente invention a pour autre objet une composition de colorant cationique, caractérisée en ce qu'elle contient un composé ionique selon l'invention. Des composés ioniques particulièrement préférés pour cette application sont ceux dans lesquels la ou les charges négatives du groupement anionique sont soit fixées à la molécule de colorant, soit elles constituent le contre-ion des charges positives du colorant. D'autres composés préférés pour cette application sont ceux dans lesquels le radical Z est un radical bivalent comprenant au moins un groupement cationique - (W=W) n-W⁺-, dans lesquels W représente un atome d'azote ou un groupement -C(R)- (R étant un radical organique) et 0≤n≤5.

La présente invention est illustrée par les exemples suivants auxquels elle n'est toutefois pas limitée.

### Exemple 1

Dans 200 ml de dichlorométhane anhydre à -20°C, sous agitation mécanique et sous argon, contenant 20,81 g (200 mmoles) d'acide malonique HOOCCH₂COOH et 41,63 g (200 mmoles) d'hexafluoroacétone trihydrate CF₃COCF₃·3H₂O (commercialisé par Aldrich), on a ajouté lentement 95,17 g (800 mmoles) de chlorure de thionyle SOCl₂ dilué par 100 ml de dichlorométhane anhydre. Lorsque l'addition a été terminée (environ 2 heures), la réaction s'est poursuivie pendant 24 heures à -20°C puis 24 heures à l'ambiante. Le solvant a alors été évaporé et le résidu repris dans 100 ml d'eau. On a alors ajouté par portions 15,2 g (110 mmoles) de carbonate de potassium anhydre K₂CO₃, puis on a recristallisé le précipité obtenu après avoir ajouté 14,91 g (200 mmoles) de chlorure de potassium anhydre KCl. Après filtration et séchage, on a récupéré 35,8 g (71% de rendement) du sel de potassium de la 2,2-trifluorométhyl-1,3-dioxolane-4,6-dione (I), ayant une pureté déterminé par RMN du proton supérieure à 98%.

On a obtenu le sel de lithium par échange ionique (métathèse) avec le chlorure de lithium dans le THF.

Par un procédé similaire mais utilisant un seul équivalent de chlorure de thionyle, on a obtenu :
- le sel de potassium de la 2-méthyl-2-trifluorométhyl-1,3-dioxolane-4,6-dione (II), en remplaçant l'hexafluoroacétone par la 1,1,1-trifluoroacétone (pureté 98% et rendement 65%),
- le sel de potassium de la 2-méthyl-2-heptafluoropropyl-1,3-dioxolane-4,6-dione (III), en remplaçant l'hexafluoroacétone par la 3,3,4,4,5,5,5-heptafluoro-2-pentanone (pureté 97% et rendement 71%), et
- le sel de potassium de la 2-phényl-2-trifluorométhyl-1,3:-dioxolane-4,6-dione (IV), en remplaçant l'hexafluoroacétone par la 2,2,2-trifluoroacétophénone (pureté 99% et rendement 76%).

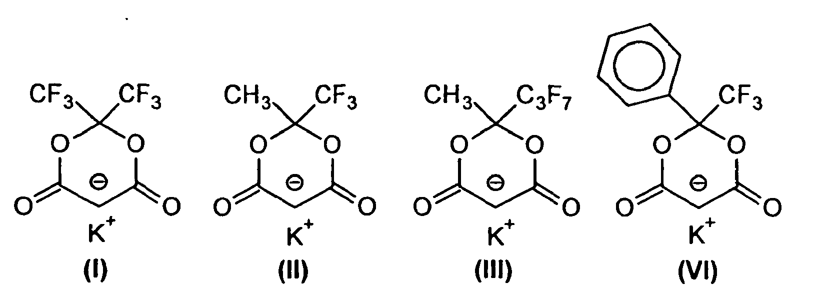

On a préparé les acides correspondants par extraction à l'éther d'une solution acide de ces sels.

On a préparé les sels de potassium des acides portant un groupement nitrile en C-5 par la procédure suivante. Dans 20 ml d'acétonitrile anhydre contenant 2,12 g (20 mmoles) de bromure de cyanogène BrCN, on a ajouté 2,87 g (20 mmoles) de chlorure d'argent AgCl, puis 20 mmoles de l'un des composés I à VI et 4,49 g (40 mmoles) de 1,4-diazabicyclo[2.2.2]octane (DABCO). Après 24 heures, le solvant a été évaporé et le résidu recristallisé dans une solution aqueuse saturée de chlorure de potassium. Ensuite, on a séché et filtré.

On a préparé les sels de potassium des acides portant un groupement trifluorométhanesulfonyle en C-5 par la procédure suivante. A 50 mmoles de l'un des composés XX, choisi parmi les composés I à VI, en solution dans 60 ml de tétrahydrofurane (THF) anhydre, à -20°C et sous agitation mécanique, on a ajouté 11,22 g (100 mmoles) de 1,4-diazabicyclo[2.2.2]octane (DABCO), puis goutte à goutte 8,43 g (100 mmoles) de chlorure de trifluorométhanesulfonyle CF₃SO₂Cl. Après 24 heures, le milieu réactionnel a été agité pendant 4 heures avec 2,12 g (50 mmoles) de chlorure de lithium anhydre. Après filtration pour éliminer le précipité d'hydrochlorure de DABCO, le solvant a été évaporé. On a récupéré quantitativement après séchage le sel de lithium d'un composé correspondant au composé XX initial, et portant une groupe trifluorométhanesulfonyle en C5.

Par une procédé similaire, on a préparé : les sels de potassium des acides portant un 4-styrènesulfonyle en C-5, en remplaçant le chlorure de trifluorométhanesulfonyle par le chlorure de 4-styrènesulfonyle (commercialisé par la société Dajac Monomers & Polymers) ; les sels de potassium des acides portant un groupement perfluorobutanesulfonyle en C-5 en remplaçant le chlorure de trifluorométhanesulfonyle par le fluorure perfluorobutane sulfonyle ; les sels de potassium des acides portant un groupement perfluorobutanesulfonyle en C-5 en remplaçant le chlorure de trifluorométhanesulfonyle par le fluorure perfluorobutane sulfonyle ; et les sels de potassium des acides portant un groupement vinylsulfonyle en C-5, en remplaçant le chlorure de trifluorométhanesulfonyle par le fluorure d'éthylènesulfonyl (commercialisé par la société ACROS).

Dans tous les cas, on a obtenu les sels de lithium par échange ionique (métathèse) entre les sels de potassium et le chlorure de lithium dans le THF.

### Exemple 2

Dans 15 ml d'acide méthanesulfonique à 0°C, on a ajouté 5,8 g (10 mmoles) du sel de potassium de la 5-perfluorobutanesulfonyl-2-phényl-2-trifluorométhyl-1,3-dioxolane-4,6-dione, obtenu à l'exemple 8, et 3,22 g (10 mmoles) de diacétate de iodosobenzène φ-I(OCOCH₃)₂ (commercialisé par Lancaster). Après 6 heures sous agitation à 0°C, le milieu réactionnel a été versé dans 100 ml d'éther, et le précipité apparu récupéré par filtration et séché. Le zwitterion obtenu : 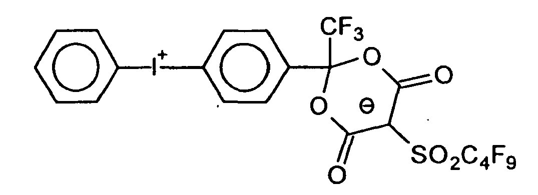 permet de libérer, sous l'effet d'un rayonnement actinique (lumière, rayons γ, faisceaux d'électrons), un acide capable d'initier une polymérisation par un mécanisme cationique. Il est soluble dans la plupart des solvants organiques usuels (tétrahydrofurane, acétonitrile, diméthylformamide, acétate d'éthyle, glymes,...) et dans les polymères solvatants aprotiques comme le polyoxyde d'éthylène. Il est aussi soluble à plus de 5% en poids dans les solvants réactifs comme le triéthylèneglycol divinyl éther.

### Exemple 3

2,8 g (10 mmoles) d'acide 4,4'-azobis(4-cyanovalérique), 3,24 g (20 mmoles) de carbonyldiimidazole et 100 mg de diméthylamino pyridine ont été mis en suspension dans 20 ml d'éther et maintenus à 0°C. Après cessation du dégagement de CO₂ (5 heures), on a ajouté 1,44 g (20 mmoles) du sel de potassium de la 2,2-trifluorométhyl-1,3-dioxolane-4,6-dione, obtenu à l'exemple 8. Le mélange a été maintenu sous agitation magnétique à 0°C pendant 24 heures. Par centrifugation, on a isolé le précipité cristallin de :

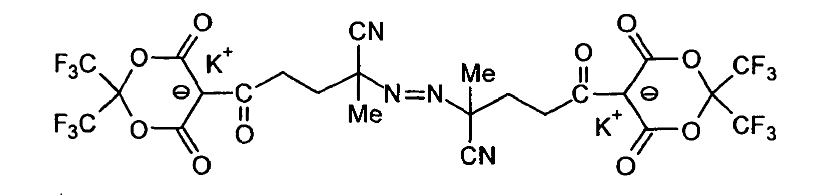

Ce sel est soluble dans la plupart des solvants organiques usuels, en particulier dans l'acétone, l'acétonitrile, l'acétate d'éthyle, le tétrahydrofurane, et dans les polymères solvatants aprotiques comme le polyoxyde d'éthylène. Il peut servir d'intiateur radicalaire non volatil pour amorcer des réactions de polymérisation ou de réticulation dès 60°C.

### Exemple 4

Une solution dans 100 ml de THF anhydre de 17 g (40 mmoles) du sel de lithium de l'acide 5-(4-styrène-sulfonyl)-2,2-trifluorométhyl-1,3-dioxolane-4,6-dione préparé comme à l'exemple 8, de 3,18 g d'acrylonitrile (60 mmoles) et de 100 mg de 1,1'-azobis(cyclohexanecarbonitrile) a été dégazée par un balayage d'argon sec. On a alors effectué sous argon la copolymérisation de l'acrylonitrile avec le dérivé du styrène en chauffant le milieu réactionnel à 60°C pendant 48 heures. Après refroidissement, la solution a été concentrée, puis le polymère récupéré par reprécipitation dans l'éther. Après filtration et séchage, on a obtenu le sel de lithium du poly(acrylonitrile-co-5-(4-styrènesulfonyl)-2,2-trifluorométhyl-1,3-dioxolane-4,6-dione) (PANS2MF).

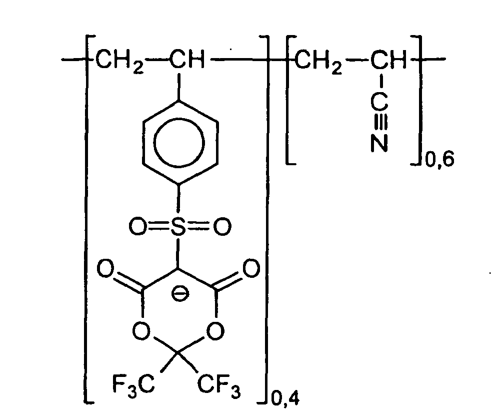

Ce polymère permet de réaliser des électrolytes polymères gélifiés à anions fixes. Il constitue la matrice permettant d'obtenir le gel et il présente les propriétés d'un polyélectrolyte.

On a préparé un électrolyte gélifié composé de 40% en poids de PANS2MF, 28% en poids de carbonate d'éthylène, 28% en poids de carbonate de propylène et 4% en poids de particules de silice (AEROSIL R 974 commercialisé par la société Degussa). Ce gel présente de bonnes propriétés mécaniques et une conductivité de 5,7.10⁻⁴ S.cm⁻¹ à 30°C. Le nombre de transport cationique de cet électrolyte a été estimé à 0,95.

On a assemblé un générateur électrochimique en utilisant ledit électrolyte gélifié, une anode composite constituée par un coke de carbone (80% en volume) mélangé avec le copolymère (PANS2MF) comme liant (20% en volume), et une cathode composite constituée par du noir de carbone (6% en volume), LiCoO₂ (75% en volume) et du copolymère (PANS2MF) comme liant (20% en volume). Ce générateur a permis d'effectuer 1000 cycles de charge/décharge entre 3 et 4,2 V en conservant une capacité supérieure à 80% de la capacité au premier cycle, lors d'un cyclage à 25°C. Il présente de très bonnes performances lors d'appel de puissance du fait de l'utilisation d'anions fixes. L'utilisation d'anions fixes a également permis d'améliorer l'évolution de la résistance d'interface.

### Exemple 5

Suivant un procédé similaire à celui utilisé dans l'exemple 4, on a synthétisé un copolymère d'acrylonitrile (97% en moles) et du sel de lithium de l'acide 5-(4-styrène-sulfonyl)-2,2-trifluorométhyl-1,3-dioxolane-4,6-dione (3% en moles).

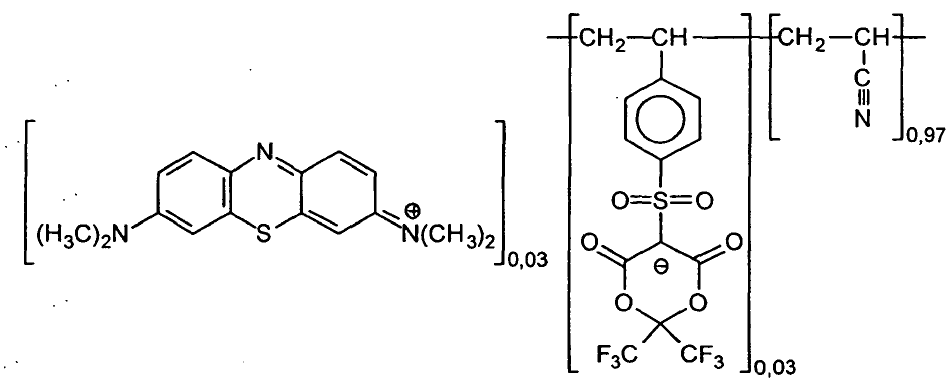

Ce copolymère, sous forme d'un sel de métal alcalin ou d'ammonium, présente des propriétés antistatiques et peut donc avantageusement remplacer les homopolymères d'acrylonitrile qui sont à ce jour largement utilisés sous forme de fibre pour le textile, mais qui ne présentent pas de propriétés antistatiques. De plus, le filage de ce copolymère est plus aisé que celui du PAN non modifié.

Le copolymère présente de très bonnes interactions avec les colorants cationiques comme le bleu de méthylène, ce qui en fait un matériau d'intérêt pour les fibres textiles colorées. La stabilité de la couleur étant nettement améliorée par rapport au copolymère classique d'acrylonitrile et de méthallylsulfonate.

### Exemple 6

A 3,2 g (25 mmoles) de 2-(3-thiényl)éthanol dans 60 ml de diméthylformamide anhydre, on a ajouté 8,4 g (25 mmoles) du sel de potassium de la 2-méthyl-2-heptafluoropropyl-1,3-dioxolane-4,6-dione, obtenu à l'exemple 1, 3,46-g de carbonate de potassium K₂CO₃ anhydre (25 mmoles) et 330 mg (1,25 mmoles) d'un éther-couronne, le 18-Crown-6 (agissant comme complexant du cation potassium). Le milieu réactionnel a alors été agité sous argon à 85°C. Après 48 heures, le milieu réactionnel a été filtré sur un verre fritté de porosité N°3, puis le solvant évaporé sous pression réduite. Après séchage, le composé a été recristallisé dans 20 ml d'eau contenant 1,86 g (25 mmoles) de chlorure de potassium KCl anhydre. Après filtration et séchage, on a récupéré le composé suivant :

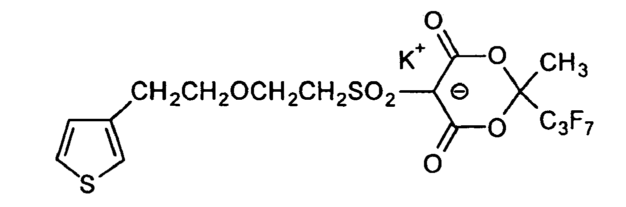

On a préparé 10 ml d'une solution 5.10⁻² M dudit composé dans l'acétonitrile et l'on a effectué une électropolymérisation dans le compartiment anodique d'une cellule électrochimique sur électrode de platine. On a obtenu un film souple conducteur de : 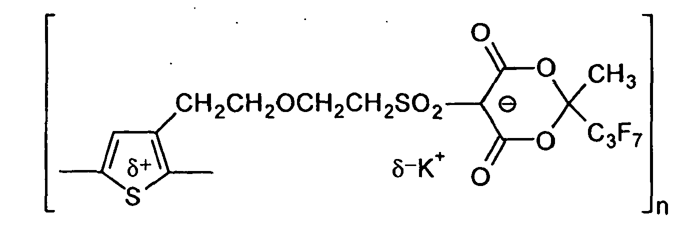 dont le dopage (oxydation) est assuré par échange de cations et d'électrons avec l'extérieur. La conductivité de ce matériau est de l'ordre de 10 S.cm⁻¹ et elle est stable à l'atmosphère ambiante et en milieu humide. L'électropolymérisation effectuée en présence de pyrrole non substitué
ou possédant des chaînons oxyéthylène en position N ou 3 donne des copolymères également stables dont le changement de couleur peut être utilisé pour la constitution de systèmes électrochromes.

### Exemple 7

Dans un réacteur chimique Parr, on a mis en solution dans 60 ml d'acétonitrile anhydre, 12,9 g (50 mmoles) du sel de lithium de la 2,2-trifluorométhyl-1,3-dioxolane-4,6-dione, obtenu à l'exemple 1, et 176 mg d'un éther-couronne, le 12-Crown-4 (agissant comme complexant du cation lithium). Après avoir fermé le réacteur, on a effectué un balayage d'argon pendant 15 min avant de l'isoler. On a alors introduit 6,41 g (50 mmoles) de dioxyde de soufre SO₂ (commercialisé par Fluka), puis, après 10 min, 9,52 g (50 mmoles) de vinyltriéthoxysilane (commercialisé par Fluka) en solution dans 20 ml d'acétonitrile anhydre. Après 6 heures à température ambiante, le réacteur a été porté à 40°C et maintenu à cette température pendant 48 heures, puis le solvant a été évaporé. Après avoir été séché sous vide, on a obtenu quantitativement le composé suivant :

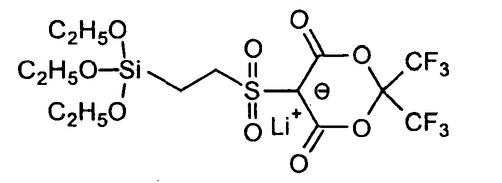

Ce sel peut former des réseaux organosiliciés par un mécanisme d'hydrolyse-polycondensation. Il peut également d'ensimer les matériaux à base de verre (fibre, vitrage,...) afin de modifier leur état de surface et en particulier de leur conférer des propriétés antistatiques. On a réalisé une solution dudit sel avec le O-[2-(triméthoxysilyl)éthyl]-O'-méthylpolyéthylène glycol de masse 5000 (commercialisé par Shearwaters Polymers) (3:1 molaire) dans un mélange eau/méthanol. Une plaque de verre décapée à l'acide nitrique puis séchée à 100°C a ensuite été trempée dans la solution pendant quelques minutes. Après rincage au méthanol et séchage, on a mesuré une conductivité de surface de 3.10⁻⁵ S(carré) suffisante pour donner des propriétés antistatiques et hydrophiles à la surface du verre.

### Exemple 8

Au cours d'une première étape, on a préparé le dilithien du ferrocène complexé par la tétraméthyléthylènediamine (TMEDA): En boîte à gants sous argon, on a placé 37 ml de TMEDA (247 mmoles) fraîchement distillé et 40 ml d'hexane anhydre dans un ballon de 1 1. On a ensuite ajouté goutte à goutte 154 ml d'une solution 1,6 M de butyllithium dans l'hexane (247 mmoles, commercialisé par Aldrich). Après 10 mn, on a ajouté goutte à goutte 18,6 g de ferrocène (100 mmoles) en solution dans 500 ml d'hexane anhydre tout en maintenant une forte agitation de la solution. Après une nuit, il est apparu des cristaux oranges dans la solution, qui ont été récupérés par filtration de la solution sur un verre fritté de porosité N°4. Après séchage sous vide, on a obtenu 28,4 g de 1,1'-dilithioferrocène·2 TMEDA (66% de rendement) que l'on a conservés sous argon.

8,61 g (20 mmoles) de ce composé dans 30 ml d'acétonitrile anhydre ont ensuite été traités par 4,89 g de 1,3-propane sultone (40 mmoles) en boîte à gants. Après 24 heures à température ambiante, on a ajouté deux gouttes de diméthylformamide dans le milieu réactionnel, puis l'on a ajouté lentement 5,08 g de chlorure d'oxalyle ClCOCOCl (40 mmoles) en solution dans 15 ml de dichlorométhane anhydre. Après 4 heures à température ambiante, on a ajouté 5 ml de pyridine et 11,61 g (40 mmoles) du sel de potassium de la 2,2-trifluorométhyl-1,3-dioxolane-4,6-dione préparé comme à l'exemple 1. La réaction s'est poursuivie pendant 24 heures, puis le milieu réactionnel a été agité 24 heures en présence de 4 g de carbonate de lithium Li₂CO₃. Après filtration et évaporation des solvants, on a récupéré après séchage le composé suivant :

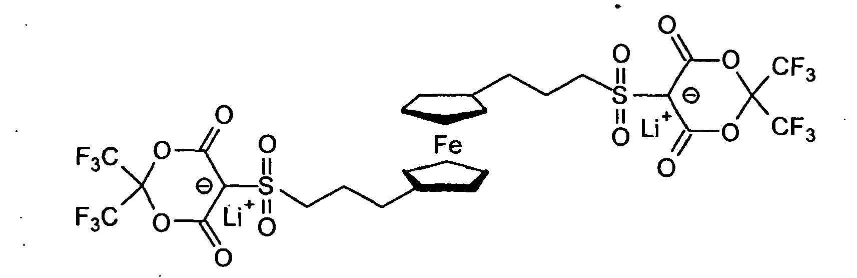

Ce sel est soluble dans la plupart des solvants organiques usuels (tétrahydrofurane, acétonitrile, diméthylformamide, acétate d'éthyle, glymes,...) et dans les polymères polaires.

Il présente un couple rédox réversible. Dans le polyoxyde d'éthylène, on a pu déterminer, sur une électrode de platine d'un diamètre de 125 µm, un potentiel réversible ≈ 3,4 V vis à vis d'une électrode de lithium.

Par dissolution dans un électrolyte liquide, gel ou polymère, il permet d'effectuer une protection en surcharge jouant ainsi un rôle de navette rédox. Il permet aussi de réaliser des systèmes électrochromes à colorants.

### Exemple 29

Le sel de lithium de l'acide 5-cyano-2,2-trifluorométhyl-1,3-dioxolane-4,6-dione, obtenu à l'exemple 1, a été testée dans des générateurs électrochimiques de technologie lithium-polymère.

Pour ledit sel, on a réalisé une batterie superposant les couches suivantes :
- un collecteur de courant en acier inoxydable ayant une épaisseur de 2 mm ;
- une cathode constituée par une pastille d'un film de matériau composite ayant une épaisseur de 89 µm et constitué par du dioxyde de vanadium (45% en volume), du noir de Shawinigan (5% en volume) et un polyoxyde d'éthylène de masse M_{w} 3.10⁵ (50% en volume) ;
- un électrolyte constitué par une pastille d'un film de polyoxyde d'éthylène de masse M_{w} = 5.10⁶ contenant ledit sel de lithium à une concentration O/Li = 15/1 ;
- une anode constituée par une feuille de lithium métallique ayant une épaisseur de 50µm ;
- un collecteur de courant analogue au collecteur précité.

Les pastilles constituant les électrodes et l'électrolyte ont été découpées en boîte à gants et empilées dans l'ordre indiqué ci-dessus. Les collecteurs ont ensuite été déposés de part et d'autre de l'empilement obtenu.

On a scellé le tout dans un boîtier de pile bouton, qui permet à la fois de protéger le générateur de l'atmosphère et d'excercer une contrainte mécanique sur les films. La batterie a alors été placée dans une enceinte sous argon installée dans une étuve à une température de 60°C. Elle a ensuite été cyclée entre 1,8 et 3,3 V à un régime de charge et de décharge de C/10 (capacité nominale chargée ou déchargée en 10 heures).

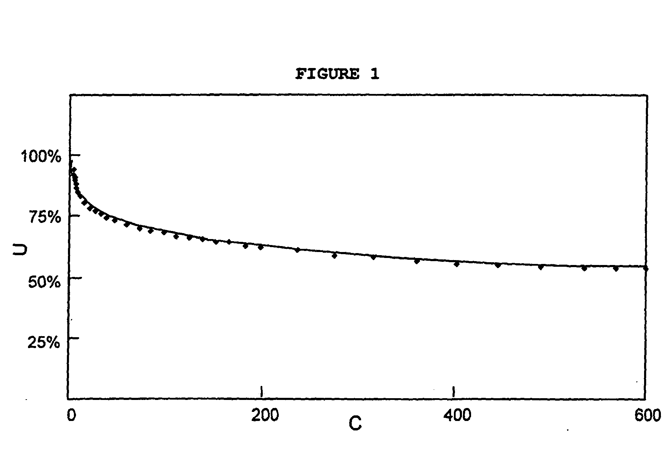

La courbe de cyclage obtenue en utilisant le sel de lithium de l'acide 5-cyano-2,2-trifluorométhyl-1,3-dioxolane-4,6-diode est similaire à celle donnée sur la figure 1. Sur cette figure, l'utilisation, U, exprimée en % est donnée en ordonnée, et le nombre de cycles C est donné en abscisse.

## Revendications

1. Composé ionique comprenant au moins une partie anionique associée à au moins une partie cationique M en nombre suffisant pour assurer la neutralité électronique de l'ensemble, **caractérisé en ce que** M est un hydroxonium, un nitrosonium NO⁺, un ammonium -NH₄⁺, un cation métallique ayant la valence m, un cation organique ayant la valence m ou un cation organométallique ayant la valence m, et **en ce que** la partie anionique est un hétérocycle aromatique répondant à la formule 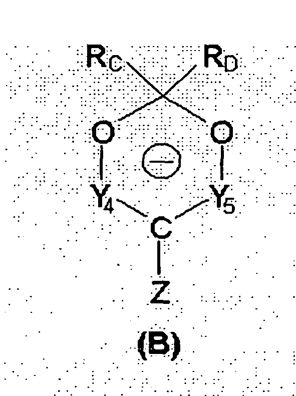 dans laquelle :
- Y₄ et Y₅ représentent chacun un groupement carbonyle ;
- Z représente un radical électro-attracteur choisi parmi les radicaux suivants :
• les radicaux R_{E}Y_{E}- ou R_{E}R_{G}PO- dans lesquels Y_{E} représente un groupement carbonyle, un groupement sulfonyle ou un groupement thionyle, et R_{E} et R_{G} représentent indépendamment l'un de l'autre un halogène ou un radical organique choisi parmi :
e) les radicaux alkyle, alkényle, oxaalkyle, oxaalkényle, azaalkyle, azaalkényle, thiaalkyle, thiaalkényle, aryle, alkylaryle, alkénylaryle, arylalkyle, arylalkényle, les radicaux alicycliques ou les radicaux aromatiques portant éventuellement au moins une chaîne latérale comprenant un hétéroatome ou comprenant éventuellement au moins un hétéroatome dans le cycle, lesdits R_{E} et R_{G} pouvant être halogénés ou perhalogénés ;
f) les radicaux alkyles ou alkényles ayant de 1 à 12 atomes de carbone et comprenant éventuellement au moins un hétéroatome O, N ou S dans la chaîne principale ou dans une chaîne latérale, et portant un groupe hydroxy, un groupe carbonyle, un groupe amine, un groupe carboxyle, un groupe isocyanate ou un groupe thioisocyanate ;
g) les radicaux aryle, arylalkyle, alkylaryle ou alkénylaryle, dans lesquels les noyaux aromatiques, éventuellement condensés, comprennent des hétéroatomes tels que l'azote, l'oxygène, le soufre ;
h) les radicaux possédant un groupement iodonium, sulfonium, oxonium, ammonium, amidinium, guanidinium, pyridinium, imidazolium, triazolium, phosphonium ou carbonium, ledit groupement jouant totalement ou partiellement le rôle du cation M ;
• -F, -Cl, -Br, -CN, -NO₂, -SCN et -N₃ ;
• -CₙF₂ₙ₊₁, -O-CₙF₂ₙ₊₁, -S-CₙF₂ₙ₊₁, -CH₂-CₙF₂ₙ₊₁, -OCF=CF₂ OU -SCF=CF₂, 1≤n≤8 ;
• les radicaux ayant une valence v au moins égale à 2 et reliant v groupes ioniques 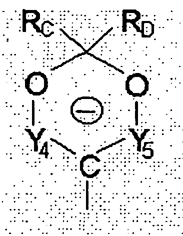
- chacun des substituants R_{C} et R_{D} représente indépendamment l'un de l'autre un radical organique monovalent ou divalent choisi parmi :
a) un alkyle, un alkényle, un oxa-alkyle, un oxa-alkényle, un aza-alkyle, un aza-alkényle, un thia-alkyle, un thia-alkényle, lesdits radicaux portant éventuellement au moins un groupe aryle ;
b) un aryle portant au moins éventuellement au moins un radical alkyle, alkényle, oxa-alkyle, oxa-alkényle, aza-alkyle, aza-alkényle, thia-alkyle ou thia-alkényle ;
c) un radical alicyclique ou un radical aromatique portant éventuellement au moins une chaîne latérale comprenant un hétéroatome ou comprenant éventuellement au moins un hétéroatome dans le cycle ;
d) un radical tel que défini ci-dessus en a), b) et c) et portant en outre des atomes d'halogène, sous la forme halogénée ou perhalogénée ;
ou fait partie d'une chaîne polymère, l'un au moins d'entre eux étant un radical perfluoré.

2. Composé selon la revendication 1, **caractérisé en ce que** le cation organique est un cation onium choisi dans le groupe constitué par les cation R₃O⁺ (oxonium), NR₄⁺ (ammonium), RC (NHR₂)₂⁺ (amidinium), C(NHR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃C⁺ (carbonium), les radicaux R représentant de manière indépendante un H ou un radical choisi dans le groupe constitué par :
- les radicaux alkyles, alkényles, oxa-alkyles, oxa-alkényles, aza-alkyles, aza-alkényles, thia-alkyles, thia-alkényles, sila-alkyles, sila-alkényles, aryles, arylalkyles, alkylaryles, alkényl-aryles, les radicaux dialkylamino et les radicaux dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore ;
étant entendu que plusieurs radicaux R peuvent former ensemble des cycles aliphatiques ou aromatiques englobant éventuellement le centre portant la charge cationique.

3. Composé selon la revendication 2, **caractérisé en ce que** le cation onium fait partie du radical Z.

4. Composé selon la revendication 2, **caractérisé en ce que** le cation onium fait partie d'une unité récurrente d'un polymère.

5. Composé selon la revendication 2, **caractérisé en ce que** le cation onium comprend un groupe hétérocyclique cationique à caractère aromatique, comportant au moins un atome d'azote alkylé dans le cycle.

6. Composé selon la revendication 5, **caractérisé en ce que** le cation onium comprend un groupe imidazolium, un groupe triazolium, un groupe pyridinium, un groupe 4-diméthylamino-pyridinium, lesdits groupes portant éventuellement un substituant sur les atomes de carbone du cycle.

7. Composé selon la revendication 2, **caractérisé en ce que** le cation onium est un groupement possédant une liaison -N=N-, -N=N⁺, un groupement sulfonium, un groupement iodonium, ou un cation arène-ferrocénium substitué ou non, éventuellement incorporé dans une trame polymérique.

8. Composé selon la revendication 7, **caractérisé en ce que** le cation onium est un cation diaryliodonium, un cation dialkylaryliodonium, un cation triarylsulfonium, un cation trialkylaryle sulfonium, ou un cation phénacyl-dialkyl sulfonium substitué ou non.

9. Composé selon la revendication 2, **caractérisé en ce que** le cation onium fait partie d'un polymère.

10. Composé selon la revendication 2, **caractérisé en ce que** le cation onium comprend un groupement 2,2'[Azobis(2-2'-imidazolinio-2-yl)propane]²⁺ ou 2,2'-Azobis(2-amidiniopropane)²⁺.

11. Composé selon la revendication 1, **caractérisé en ce que** le cation est un cation métallique choisi dans le groupe constitué par les cations de métaux alcalins, les cations de métaux alcalino-terreux, les cations de métaux de transition et les cations de métaux trivalents.

12. Composé selon la revendication 1, **caractérisé en ce que** le cation est un métallocénium, choisi dans le groupe constitué par les cations dérivés du ferrocène, du titanocène, du zirconocène, les cations indénocénium, les cations arène métallocénium, les cations des métaux de transition complexés par des liguands de type phosphine possédant éventuellement une chiralité et les cations organométalliques possédant un ou plusieurs groupements alkyles ou aryles fixés d'une manière covalente à un atome ou un groupe d'atome, lesdits cations faisant éventuellement partie d'une chaîne polymère.

13. Composé selon la revendication 1, **caractérisé en ce que** l'un des substituants R_{C} et R_{D} est choisi parmi les radicaux alkyles et les radicaux alkényles ayant de 1 à 10 atomes de carbone.

14. Composé selon la revendication 1, **caractérisé en ce que** l'un des substituants R_{C} et R_{D} est choisi parmi les radicaux alkyles ou alkényles halogénés ou perhalogénés ayant de 1 à 10 atomes de carbone.

15. Composé selon la revendication 1, **caractérisé en ce que** l'un des substituants R_{C} et R_{D} est choisi parmi les radicaux oxa-alkyles ou oxa-alkényles ayant de 1 à 10 atomes de carbone.

16. Composé selon la revendication 1, **caractérisé en ce que** Z est un radical R_{E}SO₂-.

17. Composé selon la revendication 1, **caractérisé en ce que** R_{E} ou R_{G} comporte un groupement susceptible de piéger les radicaux libres, choisi parmi les phénols encombrés et les quinones.

18. Composé selon la revendication 1, **caractérisé en ce que** R_{E} ou R_{G} comporte un dipôle dissociant choisi parmi les fonctions amide, sulfonamide et nitrile.

19. Composé selon la revendication 1, **caractérisé en ce que** R_{E} ou R_{G} comporte un couple rédox choisi parmi un groupe disulfure, un groupe thioamide, un groupe ferrocène, un groupe phénothiazine, un groupe bis(dialkylaminoaryle), un groupe nitroxyde ou un groupe imide aromatique.

20. Composé selon la revendication 1, **caractérisé en ce que** R_{E}-Y_{E}- ou R_{E}R_{G}PO- est optiquement actif.

21. Composé selon la revendication 1, **caractérisé en ce que** Z est partie d'une unité récurrente d'une chaîne polymère.

22. Composé selon la revendication 1, **caractérisé en ce que** R_{E} ou R_{G} est partie d'un radical poly(oxyalkylène) ou d'un radical polystyrène.

23. Matériau à conduction ionique comprenant un composé ionique en solution dans un solvant, **caractérisé en ce que** le composé ionique est un composé selon la revendication 1.

24. Matériau à conduction ionique selon la revendication 23, **caractérisé en ce que** le cation du composé ionique est l'ammonium, un cation dérivé d'un métal, ou un cation organique ammonium substitué, imidazolium, triazolium, pyridinium ou 4-diméthylamino-pyridinium, lesdits cations organiques portant éventuellement un substituant sur les atomes de carbone du cycle.

25. Matériau à conduction ionique selon la revendication 23, **caractérisé en ce que** le composé ionique est tel que l'un au moins des substituants R_{C}, R_{D}, R_{E} ou R_{G} est un groupe alkyle, un groupe aryle, un groupe alkylaryle ou un groupe arylalkyle, ou un groupe alkyle ou alkényle qui contient au moins un hétéroatome choisi parmi O, N et S et/ou un groupe hydroxy, un groupe carbonyle, un groupe amine, un groupe carboxyle.

26. Matériau à conduction ionique selon la revendication 23, **caractérisé en ce que** l'un au moins des substituants R_{E} ou R_{G} est choisi parmi les radicaux aryle, arylalkyle, alkylaryle ou alkénylaryle, dans lesquels les chaînes latérales et/ou les noyaux aromatiques comprennent des hétéroatomes tels que l'azote, l'oxygène, le soufre.

27. Matériau à conduction ionique selon la revendication 23, **caractérisé en ce que** que le substituant Z est choisi dans le groupe constitué par -OCₙF₂ₙ₊₁, -OC₂F₄H, -SCₙF₂ₙ₊₁, -SC₂F₄H, -OCF=CF₂, -SCF=CF₂, n étant un nombre entier de 1 à 8.

28. Matériau à conduction ionique selon la revendication 23, **caractérisé en ce que** le substituant Z représente une unité récurrente d'un polymère.

29. Matériau à conduction ionique selon la revendication 23, **caractérisé en ce que** le solvant est soit un solvant liquide aprotique, choisi parmi les éthers linéaires et les éthers cycliques, les esters, les nitriles, les dérivés nitrés, les amides, les sulfones, les sulfolanes, les sulfamides et les hydrocarbures partiellement halogénés, soit un polymère polaire, soit un de leurs mélanges.

30. Matériau à conduction ionique selon la revendication 29, **caractérisé en ce que** le solvant est un polymère solvatant, réticulé ou non, portant ou non des groupements ioniques greffés.

31. Matériau à conduction ionique selon la revendication 30, **caractérisé en ce que** le polymère solvatant est choisi parmi les polyéthers de structure linéaire, peigne ou à blocs, formant ou non un réseau, à base de poly(oxyde d'éthylène), les copolymères contenant le motif oxyde d'éthylène ou oxyde de propylène ou allylglycidyléther, les polyphosphazènes, les réseaux réticulés à base de polyéthylène glycol réticulé par des isocyanates, les réseaux obtenus par polycondensation et portant des groupements qui permettent l'incorporation de groupements réticulables, et les copolymères à blocs dans lesquels certains blocs portent des fonctions qui ont des propriétés rédox.

32. Matériau à conduction ionique selon la revendication 23, **caractérisé en ce que** le solvant est constitué essentiellement par un solvant liquide aprotique et un solvant polymère polaire comprenant des unités contenant au moins un hétéroatome choisi parmi le soufre, l'oxygène, l'azote et le fluor.

33. Matériau à conduction ionique selon la revendication 32, **caractérisé en ce que** le polymère polaire contient principalement des unités dérivées de l'acrylonitrile, du fluorure de vinylidène, de la N-vinylpyrrolidone ou du méthacrylate de méthyle.

34. Matériau à conduction ionique selon la revendication 23, **caractérisé en ce qu'**il contient en outre au moins un second sel et/ou une charge minérale ou organique sous forme de poudre ou de fibres.

35. Générateur électrochimique comprenant une électrode négative et une électrode positive séparées par un électrolyte, **caractérisé en ce que** l'électrolyte est un matériau selon l'une des revendications 23 à 34.

36. Générateur selon la revendication 35, **caractérisé en ce que** l'électrode négative est constituée par du lithium métallique, ou par l'un de ses alliages, éventuellement sous forme de dispersion manométrique dans de l'oxyde de lithium, ou par un nitrure double de lithium et d'un métal de transition, ou par un oxyde ayant pour formule générale Li_{1+y+x/3}Ti_{2-x/3}O₄ (0 ≤ x ≤1, 0 ≤ y ≤ 1), ou par le carbone et les produit carbonés issus de la pyrolyse de matières organiques.

37. Générateur selon la revendication 35,**caractérisé en ce que** l'électrode positive est choisie parmi les oxydes de vanadium VOₓ (2 ≤ x ≤ 2,5), LiV₃O₈, Li_{y}Ni₁₋ₓCoₓO₂, (0 ≤ x ≤ 1 ; 0 ≤ y ≤ 1), les spinelles de manganèse Li_{y}Mn₁₋ₓMₓO₂ (M = Cr, Al, V, Ni, 0 ≤ x ≤ 0,5 ; 0 ≤ y ≤ 2), les polydisulfures organiques, FeS, FeS₂, le sulfate de fer Fe₂ (SO₄) ₃, les phosphates et phosphosilicates de fer et de lithium de structure olivine, ou leurs produits de substitution du fer par le manganèse, utilisés seuls ou en mélanges.

38. Générateur selon la revendication 35, **caractérisé en ce que** le collecteur de la cathode est en aluminium.

39. Supercapacité utilisant au moins une électrode de carbone à haute surface spécifique, ou au moins une électrode contenant un polymère rédox, dans laquelle l'électrolyte est un matériau selon l'une des revendications 23 à 34.

40. Utilisation d'un matériau selon l'une des revendications 23 à 34 pour le dopage p ou n d'un polymère à conduction électronique.

41. Dispositif électrochrome, dans lequel l'électrolyte est un matériau selon l'une des revendications 23 à 34.

42. Procédé de polymérisation ou de réticulation de monomères ou de prépolymères capables de réagir par voie cationique, **caractérisé en ce que** l'on utilise un composé selon la revendication 1 comme photoinitiateur source d'acide catalysant la réaction.

43. Procédé selon la revendication 42, **caractérisé en ce que** l'on utilise un composé ionique dont le cation est un groupement possédant une liaison -N=N⁺, -N=N- un groupement sulfonium, un groupement iodonium, ou un cation arène-ferrocénium substitué ou non, éventuellement incorporé dans une trame polymérique.

44. Procédé selon la revendication 42, **caractérisé en ce que** les monomères sont choisis dans le groupe constitué par les composés qui comportent une fonction éther cyclique, une fonction thioéther cyclique ou une fonction amine cyclique, les composés vinyliques, les éthers vinyliques, les oxazolines, les lactones et les lactames.

45. Procédé selon la revendication 42,**caractérisé en ce que** le prépolymère est choisi dans le groupe constitué par les composés dans lesquels des groupements époxy sont portés par une chaîne aliphatique, une chaîne aromatique, ou une chaîne hérérocyclique.

46. Procédé selon la revendication 42, **caractérisé en ce qu'**il consiste à mélanger le photoinitiateur avec au moins un monomère ou prépolymère capable de polymériser par voie cationique, et à soumettre le mélange obtenu à un rayonnement actinique, y compris un rayonnement β.

47. Procédé selon la revendication 42, **caractérisé en ce que** le mélange réactionnel est soumis au rayonnement après avoir été mis sous forme d'une couche mince.

48. Procédé selon la revendication 42, **caractérisé en ce que** le photoinitiateur est utilisé sous forme d'une solution dans un solvant inerte vis à vis de la réaction de polymérisation.

49. Procédé selon la revendication 48, **caractérisé en ce que** le solvant inerte est choisi dans le groupe constitué par l'acétone, la méthyl-éthyl cétone, l'acétonitrile, le carbonate de propylène, la g-butyrolactone, les éther-esters des mono-, di-, tri- éthylène ou propylène glycols, les éther-alcools des mono-, di-, tri- éthylène ou propylène glycols, les esters de l'acide phtalique ou de l'acide citrique.

50. Procédé selon la revendication 42, **caractérisé en ce que** la réaction est effectuée en présence d'un solvant ou d'un diluant constitué par un composé réactif vis à vis de la polymérisation.

51. Procédé selon la revendication 42, **caractérisé en ce que** le composé réactif est choisi dans le groupe constitué par les mono et di éthers vinyliques des mono-, di-, tri-, tétra- éthylène ou propylène glycols, le trivinyl éther triméthylolpropane et le divinyléther du diméthanolcyclohexane, la N-vinylpyrolidone, le 2-propényléther du carbonate de propylène.

52. Procédé selon la revendication 42,**caractérisé en ce que** l'on ajoute un photosensibilisateur au milieu réactionnel.

53. Procédé selon la revendication 52, **caractérisé en ce que** le photosensibilisateur est choisi dans le groupe constitué par l'anthracène, le diphényl-9,10-anthracène, le pérylène, la phénothiazine, le tétracène, la xanthone, la thioxanthone, l'isopropylthioxantone, l'acétophénone, la benzophénone, les 1,3,5-triaryl-2-pyrazolines et leurs dérivés de substitution sur les noyaux aromatiques par des radicaux alkyles, oxa- ou aza-alkyles.

54. Procédé selon la revendication 42, **caractérisé en ce que** le mélange réactionnel contient en outre au moins un monomère ou prépolymère capable de polymériser par voie radicalaire et un composé capable de libérer un amorceur de polymérisation radicalaire sous l'effet du rayonnement actinique ou du rayonnement β ou sous l'action de la chaleur.

55. Procédé de modification des propriétés de solubilité d'un polymère possédant des groupements sensibles aux acides, **caractérisé en ce qu'**il consiste à soumettre ledit polymère à un rayonnement actinique ou un rayonnement β, en présence d'un composé selon la revendication 1.

56. Procédé selon la revendication 55, **caractérisé en ce que** le polymère contient des motifs ester ou des motifs aryléther dérivés d'alcool tertiaire.

57. Procédé selon la revendication 56, **caractérisé en ce que** le polymère est choisi dans le groupe constitué par les polyacrylates de tertiobutyle, les polyitaconates de tertiobutyle ou de tertio-amyle, le poly(tertiobutoxycarbonyloxystyrène), le poly(tertiobutoxystyrène).

58. Procédé selon la revendication 55, **caractérisé en ce qu'**il est mis en oeuvre pour l'amplification chimique de photorésists.

59. Composition de colorant cationique, **caractérisée en ce qu'**elle contient un composé selon la revendication 1.

60. Composition de colorant cationique selon la revendication 59, **caractérisé en ce que** la ou les charges négatives du groupement anionique sont soit fixées à la molécule de colorant, soit elles constituent le contre-ion des charges positives du colorant.

61. Utilisation d'un composé selon la revendication 1 en tant que catalyseur dans les réactions de Friedel-Crafts, les réactions de Diels et Alder, les réactions d'aldolisation, les additions de Michael, les réactions d'allylation, les réactions de couplage pinacolique, les réactions de glycosilation, les réactions d'ouvertures de cycles des oxétanes, les réactions de méthathèse des alcènes, les polymérisations de type Ziegler-Natta, les polymérisations du type méthathèse par ouverture de cycle et les polymérisations du type métathèse de diènes acycliques.

62. Utilisation selon la revendication 61, **caractérisée en ce que** le cation du composé est choisi parmi le lithium, le magnésium, le cuivre, le zinc, l'étain, les métaux trivalents, y compris les terres rares, les platinoïdes, et les cations organométalliques.

63. Utilisation d'un composé selon la revendication 6, comme solvant pour effectuer des réactions chimiques, photochimiques, électrochimiques, photoélectrochimiques, ledit composé étant utilisé au dessus de son point de fusion.

64. Matériau à conduction électronique, **caractérisé en ce qu'**il contient un composé selon la revendication 1.

65. Matériau à conduction électronique selon la revendication 64, **caractérisé en ce que**, dans le composé ionique, au moins l'un des substituants de l'hétérocycle aromatique anionique contient une chaîne alkyle ayant de 6 à 20 atomes de carbone.

66. Matériau à conduction électronique selon la revendication 64, **caractérisé en ce que** la partie cationique du composé ionique est un polycation constitué par un polymère conjugué dopé "p".

## Claims

1. An ionic compound comprising at least one anionic part associated to at least one cationic part M in sufficient number to ensure an electronic neutrality to the compound, **characterized in that** M is an hydroxonium, a nitrosonium NO⁺, an ammonium --NH₄⁺, a metallic cation having a valence m, an organic cation having a valence m or an organometallic cation having a valence m, and **in that** the anionic part is an aromatic heterocycle corresponding to formula 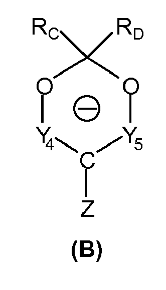 in which:
- Y₄ and Y₅ represent each a carbonyl group;
- Z represents an electroattractor radical selected from the following radicals:
• R_{E}Y_{E}- and R_{E}R_{G}PO- radicals wherein Y_{E} represents a carbonyl group, a sulfonyl group or a thionyl group, and R_{E} and R_{G} represent independently a halogen or an organic radical selected from
e) alkyl, alkenyl, oxaalkyl, oxaalkenyl, azaalkyl, azaalkenyl, thiaalkyl, thiaalkenyl, aryl, alkylaryl, alkenylaryl, arylalkyl, and arylalkenyl radicals, alicyclic or aromatic radical which may have at least one side chain comprising a heteroatom or in the ring, wherein said R_{E} and R_{G} may be halogenated or perhalogenated ;
f) alkyl or alkenyl radicals having 1 to 12 carbon atoms and comprising optionally at least one heteroatom O, N or S in the main chain or in a side chain, and having a hydroxy group, a carbonyl group, an amine group, a carboxyl group, an isocyanate group or a thioisocyanate group;
g) aryl, arylalkyl, alkylaryl or alkenylaryl groups, wherein the aromatic nuclei, which may be condensed, comprise heteroatom's such as nitrogen, oxygen and sulfur;
h) radicals comprising iodonium, sulfonium, oxonium, ammonium, amidinium, guanidinium, pyridinium, imidazolium, triazolium, phosphonium or carbonium groups, said groups acting completely or partly as the cation M ;
• -F, -Cl, -Br, -CN, -NO₂, -SCN et -N₃ ;
• -CₙF₂ₙ₊₁, -O-CₙF₂ₙ₊₁, -S-CₙF₂ₙ₊₁, -CH₂-CₙF₂ₙ₊₁, -OCF=CF₂ ou -SCF=CF₂, 1≤n≤8 ;
• radicals having a valence v at least equal to 2 et bonding v ionic groups 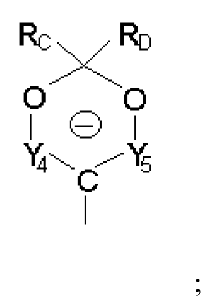
- each substituent R_{c} and R_{D} represents independently a monovalent or divalent organic radical selected from :
a) an alkyl, an alkenyl, an oxa-alkyl, an oxa-alkenyl, an aza-alkyl, an aza-alkenyl, a thia-alkyl, a thia-alkenyl, said radicals carrying at least one aryl group;
b) an aryl carrying at least one radical as defined in a);
c) an alicyclic radical or an aromatic radical optionally carrying at least one lateral chain comprising a heteroatom or optionally comprising at least one heteroatom in the cycle;
d) a radical as defined above in a), b) and c) and additionally carrying halogen atoms, in halogenated or perhalogenated form ;
or is part of a polymer chain, at least one being perfluorinated.

2. An ionic compound according to claim, **characterized in that** the organic cation is selected from a group consisting of cations R₃O⁺ (oxonium), NR₄⁺ (ammonium), RC(NHR₂)₂⁺ (amidinium), C(NHR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃ C⁺ (carbonium), the radicals R independently representing an H or a radical selected from the group consisting of:
- alkyl, alkenyl, oxa-alkyl, oxa-alkenyl, aza-alkyl, aza-alkenyl, thia-alkyl, thia-alkenyl, sila-alkyl, sila-alkenyl, aryl, arylalkyl, alkylaryl, alkenyl-aryl, dialkylamino and dialkylazo radicals;
- cyclic or heterocyclic radicals optionally comprising at least one side chain comprising heteroatoms such as oxygen, nitrogen, sulfur;
- cyclic or heterocyclic radicals optionally comprising heteroatoms in the aromatic nucleus;
- groups comprising a plurality of aromatic or heterocyclic nuclei, condensed or non-condensed, optionally containing at least one nitrogen, oxygen, sulfur or phosphorus atom;
with the proviso that a plurality of radicals R may together form aliphatic or aromatic cycles optionally enclosing the center carrying the cationic charge.

3. An ionic compound according to claim 2, **characterized in that** the onium cation is part of the radical Z.

4. An ionic compound according to claim 2, **characterized in that** the onium cation is part of a repeat unit of a polymer.

5. An ionic compound according to claim 2, **characterized in that** the onium cation comprises a cationic heterocyclic aromatic group, which comprises at least one alkylated nitrogen atom in the cyclic chain.

6. An ionic compound according to claim 5, **characterized in that** the onium cation comprises an imidazolium group, a triazolium group, a pyridinium group, a 4-dimethyl-amino-pyridinium group, said groups optionally carrying a substituent on the carbon atoms of the cycle.

7. An ionic compound according to claim 2, **characterized in that** the onium cation is a group which has a --N=N-- bond, a --N=N⁺ bond, a sulfonium group, an iodonium group, or a substituted or non-substituted arene-ferrocenium cation, optionally incorporated in a polymeric network.

8. An ionic compound according to claim 7, **characterized in that** the onium cation is a diaryliodonium cation, a dialkylaryliodonium cation, a triarylsulfonium cation, a trialkylarylsulfonium cation or a substituted or non substituted phenacyl-dialkyl sulfonium cation.

9. An ionic compound according to claim 2, **characterized in that** the onium cation is part of a polymer.

10. An ionic compound according to claim 2, **characterized in that** the onium cation comprises a 2,2'[Azobis(2-2'-imidazolinio-2-yl)propane]²⁺ or a 2,2'-Azobis(2-amidinio-propane)2⁺ group.

11. An ionic compound according to claim 1, **characterized in that** the cation is a metallic cation selected from the group consisting of cations of alkali metals, cations of alkali earth metals, cations of transition metals, cations of trivalent metals.

12. An ionic compound according to claim 1, **characterized in that** cation is a metallocenium, selected from the groups consisting of cations derived from ferrocene, titanocene or zirconocene, an indenocenium or a metallocenium arene, cations of transition metals complexed with phosphine ligands possibly having a chirality, organometallic cations having one or more alkyl or aryl groups covalently fixed to an atom or a group of atoms, such as methylzinc, phenylmercury, trialkyltin or trialkyllead cations wherein the organometallic cation may be part of a polymer chain.

13. An ionic compound according to claim 1, **characterized in that** one of the R_{c} and R_{D} substituents is selected from alkyl or alkenyl radicals having 1 to 10 carbon atoms.

14. An ionic compound according to claim 1, **characterized in that** one of the R_{c} and R_{D} substituents is selected from halogenated and perhalogenated alkyl or alkenyl radicals having 1 to 10 carbon atoms

15. An ionic compound according to claim 1, **characterized in that** one of the R_{c} and R_{D} substituents is selected from oxa-alkyl or oxa-alkenyl radicals having 1 to 10 carbon atoms

16. An ionic compound according to claim 1, **characterized in that** Z is a R_{E}SO₂- radical.

17. An ionic compound according to claim 1, **characterized in that** R_{E} or R_{G} includes a group capable of trapping free radicals, selected from hindered phenols and quinones.

18. An ionic compound according to claim 1, **characterized in that** R_{E} or R_{G} comprises a dissociating dipole selected from amide, sulfonamide and nitrile groups.

19. An ionic compound according to claim 1, **characterized in that** R_{E} or R_{G} comprises a redox couple selected from disulfide, thioamide, ferrocene, phenothiazine, bis(dialkylaminoaryl), nitroxide and aromatic imide groups

20. An ionic compound according to claims 1, **characterized in that** R_{E}-Y_{E} -- or R_{E} R_{G} PO-- is optically active.

21. An ionic compound according to claim 1, **characterized in that** Z is part of a repeat unit of a polymer chain.

22. An ionic compound according to claim 1, **characterized in that** R_{E} or R_{G} is part of a poly(oxyalkylene) radical or of a polystyrene radical.

23. Ionically conductive material comprising an ionic compound in solution in a solvent, **characterized in that** the ionic compound is a compound according to claim 1.

24. An ionically conductive material according to claim 23, **characterized in that** the cation of the ionic compound is ammonium, a cation derived from a metal, or an organic cation selected from substituted ammonium, imidazolium, triazolium, pyridinium or 4-diméthylamino-pyridinium, said organic cations having optionally a substituent on the carbon atoms of the cycle.

25. An ionically conductive material according to claim 23, **characterized in that** at least one of the substituents R_{C}, R_{D}, R_{E} or R_{G} is an alkyl group, an aryl group, an alkylaryl group or an arylalkyl group, or an alkyl or alkenyl group having at least one heteroatom selected from O, N and S and/or a hydroxy group, a carbonyl group, an amine group or a carboxyl group.

26. An ionically conductive material according to claim 23, **characterized in that** at least one of the substituents R_{E} or R_{G} is selected from aryl, arylalkyl, alkylaryl or alkenylaryl radicals wherein the side chains and/or the aromatic nuclei comprise heteroatoms such as nitrogen, oxygene, sulfur.

27. An ionically conductive material according to claim 23, **characterized in that** the substituent Z is selected from the group consisting of -OCₙF₂ₙ₊₁, -OC₂F₄H, -SCₙF₂ₙ₊₁, -SC₂F₄H, -OCF=CF₂, -SCF=CF₂, n being a number from 1 to 8.

28. An ionically conductive material according to claim 23, **characterized in that** the substituent Z is a repeat unit of a polymer.

29. An ionically conductive material according to claim 23, **characterized in that** the solvent is either an aprotic liquid solvent selected from linear ethers and cyclic ethers, esters, nitriles, nitro derivatives, amides, sulfones, sulfolanes, sulfamides and partially halogenated hydrocarbons, or a polar polymer, or a mixture thereof.

30. An ionically conductive material according to claim 23, **characterized in that** the solvent is a cross-linked or non-cross-linked solvating polymer, which may carry grafted ionic groups.

31. An ionically conductive material according to claim 30, **characterized in that** the solvating polymer is selected from polyethers of linear structure, comb or blocks, which may form a network, based on poly(ethylene oxide), or copolymers containing the unit ethylene oxide or propylene oxide or allylglycidylether, polyphosphazenes, cross-linked networks based on polyethylene glycol cross-linked with isocyanates or networks obtained by polycondensation and carrying groups which enable the incorporation of cross-linkable groups, block copolymers in which certain blocks carry functions which have redox properties.

32. Ionically conductive material according to claim 23, **characterized in that** the solvent consists essentially of an aprotic liquid solvent and a polar polymer solvent comprising units containing at least one heteroatom selected from sulfur, oxygen, nitrogen and fluorine.

33. Ionically conductive material according to claim 32, **characterized in that** the polar polymer mainly contains units derived from acrylonitrile, vinylidene fluoride, N-vinylpyrrolidone or methyl methacrylate.

34. An ionically conductive material according to claim 23, **characterized in that** it further contains at least one second salt and/or a organic or inorganic filler in the form of a powder or of fibers.

35. Electrochemical generator comprising a negative electrode and a positive electrode separated by an electrolyte, **characterized in that** the electrolyte is an ionically conductive material according to any of claims 23 to 34.

36. A generator according to claim 35, **characterized in that** the negative electrode consists of metallic lithium, or an alloy thereof, optionally in the form of nanometric dispersion in lithium oxide, or a double nitride of lithium and a transition metal, or an oxide with low potential having the general formula Li₁+y+x/3 Ti₂-x/3 O₄ (0≤x≤1, 0≤y≤1), or carbon and carbonated products produced by pyrolysis of organic material.

37. Generator according to claim 35, **characterized in that** the positive electrode is selected from vanadium oxides VOₓ (2≤x≤2,5), LiV₃O₈, LiyNi₁₋ₓCoₓO₂, (0≤x≤1; 0≤y≤1), spinels if manganese Li_{y} Mn₁₋ₓMₓO₂ (M=Cr, Al, V, Ni, 0≤x≤0,5; 0≤y≤2), organic polydisulfides, FeS, FeS₂, iron sulfate Fe₂(SO₄)₃, phosphates and phosphosilicates of iron and lithium olivine structure, or substituted products wherein iron is replaced by manganese, used alone or in mixtures.

38. Generator according to claim 35, **characterized in that** the collector of the cathode is made of aluminum.

39. A supercapacitor utilizing at least one carbon electrode with high specific surface, or at least one electrode containing a redox polymer, in which the electrolyte is a material according to any of claims 23 to 34.

40. Use of a material according to any of claims 23 to 34 for p or n doping a electronically conducting polymer.

41. Electrochrome device, in which the electrolyte is a material according to any of claims 23 to 34.

42. A method for polymerization or crosslinking monomers or prepolymers capable of reacting cationically, **characterized in that** a compound according to claim 1 is used as a photo initiator source of an acid which catalyzes the reaction

43. The method of claim 42, **characterized in that** an ionic compound is used, wherein the cation is a group having a -N=N⁺, -N=N- bond, a sulfonium group, an iodonium group or a substituted or unsubstituted arene-ferrocenium cation, optionally incorporated in a polymer network.

44. The method of claim 42, **characterized in that** the monomers are selected from the group consisting of compounds having a cyclic ether function, a cyclic thioether function or cyclic amine function, vinyl compounds, vinyl ethers, oxazolines, lactones and lactames

45. The method of claim 42, **characterized in that** the prepolymer is selected from the group consisting of compounds in which epoxy groups are carried by an aliphatic chain, an aromatic chain, or a heterocyclic chain.

46. The method of claim 42, **characterized in that** it consists in mixing the photo initiator with at least one monomer or prepolymer capable of cationic polymerization, and subjecting the mixture obtained to actinic radiation or β radiation.

47. The method of claim 42, **characterized in that** the reaction mixture is subjected to radiation after having been formed into a thin layer.

48. The method of claim 42, **characterized in that** the photo initiator is used in the form of a solution in a solvent which is inert for the polymerization reaction.

49. The method of claim 42, **characterized in that** the inert solvent is selected from the group consisting of acetone, methyl-ethyl ketone, acetonitrile, propylene carbonate, γ-butyrolactone, ether-esters of mono-, di-, tri-ethylene or propylene glycols, ether-alcohols of mono-, di-, tri-ethylene or propylene glycols, esters of phthalic acid or citric acid.

50. The method of claim 42, **characterized in that** the reaction is carried out in the presence of a solvent or a diluent consisting of a compound which is reactive toward the polymerization.

51. The method of claim 42, **characterized in that** the reactive solvent is selected from mono and divinyl ethers of mono-, di-, tri-, tetra-ethylene and propylene glycols, trimethylolpropane trivinyl ether and dimethanol-cyclohexane divinylether, N-vinylpyrolidone, and propylene carbonate 2-propenylether

52. The method of claim 42, **characterized in that** a photo sensitizer is added to the reaction mixture.

53. The method of claim 52, **characterized in that** the photo sensitizer is selected from the group consisting of anthracene, diphenyl-9,10-anthracene, perylene, phenothiazine, tetracene, xanthone, thioxanthone, isopropylthioxantone, acetophenone, benzophenone, 1,3,5-triaryl-2-pyrazolines and derivatives thereof obtained by substitution on the aromatic nuclei by alkyl, oxa- or aza-alkyl groups.

54. The method of claim 42, **characterized in that** the reaction mixture further contains at least one monomer or prepolymer capable of free radical polymerizing and a compound capable of providing a free radical polymerization initiator under an actinic radiation, a β-radiation or upon heating.

55. A method for modifying the solubility properties of a polymer having groups which are sensitive to acids, **characterized in that** said polymer is subjected to actinic radiation, or β-radiation, in the presence of a compound according to claim 1.

56. The method of claim 55, **characterized in that** the polymer has ester units or arylether units derived from a tertiary alcohol.

57. The method of claim 56, **characterized in that** the polymer is selected from the group consisting of tertiobutyl polyacrylates, tertiobutyl or tertioamylpolyitaconates, poly(tertiobutoxycarbonyloxystyrene), poly(tertiobutoxystyrene).

58. The method according to claim 55, which is implemented for chemical amplification of photoresists.

59. Composition of cationic colouring material, **characterized in that** it contains a compound according to claim 1.

60. The composition of cationic colouring material according to claim 59, **characterized in that** the negative charge(s) of the anionic group are either fixed to the molecule of the colouring material, or they constitute the counter-ion of the positive charges of the colouring material.

61. Use of a compound according to claim 1, as the catalyst in Friedel-Crafts reactions, Diels and Alder reactions, aldolization reactions, additions of Michael, reactions of allylation, reactions of pinacolic coupling, reactions of allylation, reactions of cyclic openings of oxetane, reactions of metathesis of alkenes, polymerizations of Ziegler-Natta type, polymerizations of metathesis type by cycle opening and polymerizations of the metathesis type of acyclic dienes.

62. Use according to claim 61, **characterized in that** the cation of the compound is selected from lithium, magnesium, copper, tin, zinc, trivalent metals, including rare earths, platinoids and organometallic cations.

63. Use of a compound according to claim 6, as the solvent for chemical, photochemical, electrochemical and photoelectrochemical reactions, said compound being used at a temperature lower than the melting temperature.

64. Electronically conducting material, **characterized in that** it contains a compound according to claim 1.

65. The electronically conducting material of claim 63, **characterized in that** at least one of the substituents of the anionic aromatic heterocycle contains an alkyl chain having 6 to 20 carbon atoms.

66. The electronically conducting material of claim 64, **characterized in that** the cationic part of the ionic compound is a polycation consisting of a "p" doped conjugated polymer.

## Patentansprüche

1. Ionische Verbindung, umfassend zumindest einen anionischen Anteil, der mit zumindest einem kationischen Anteil M in ausreichender Anzahl verbunden ist, um insgesamt elektrische Neutralität zu gewährleisten, **dadurch gekennzeichnet, dass** M Hydroxonium, Nitrosonium NO⁺, Ammonium -NH₄⁺, ein Metallkation mit der Wertigkeit m, ein organisches Kation mit der Wertigkeit m oder ein Organometall-Kation mit der Wertigkeit m ist und dass der anionische Anteil ein aromatischer Heterozyklus der Formel 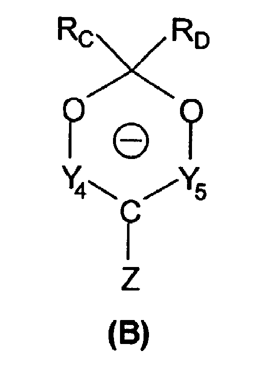 ist, worin:
- Y₄ und Y₅ jeweils für eine Carbonylgruppe stehen;
- Z für einen aus folgenden Resten ausgewählten, Elektronen anziehenden Rest steht:
• den Resten R_{E}Y_{E}- oder R_{E}R_{G}PO-, worin Y_{E} für eine Carbonylgruppe, eine Sulfonylgruppe oder Thionylgruppe steht und R_{E} und R_{G} unabhängig voneinander für ein Halogen oder einen aus folgenden ausgewählten organischen Rest stehen:
e) Alkyl-, Alkenyl-, Oxaalkyl-, Oxaalkenyl-, Azaalkyl-, Azaalkenyl, Thiaalkyl-, Thiaalkenyl-, Aryl-, Alkylaryl-, Alkenylaryl-, Arylalkyl-, Arylalkenylresten, alizyklischen Resten oder aromatischen Resten, die gegebenenfalls zumindest eine Seitenkette, die ein Heteroatom umfasst, oder gegebenenfalls zumindest ein Heteroatom im Ring umfassen, wobei R_{E} und R_{G} gegebenenfalls halogeniert oder perhalogeniert sind;
f) Alkyl- oder Alkenylresten mit 1 bis 12 Kohlenstoffatomen, die gegebenenfalls zumindest ein O-, N- oder S-Heteroatom in der Haupt- oder in einer Seitenkette umfassen und eine Hydroxygruppe, Carbonylgruppe, Aminogruppe, Carboxylgruppe, lsocyanatgruppe oder Thioisocyanatgruppe aufweisen;
g) Aryl-, Arylalkyl-, Alkylaryl- oder Alkenylaryl-Resten, in denen die, gegebenenfalls kondensierten, aromatischen Kerne Heteroatome, wie z.B. Stickstoff, Sauerstoff und Schwefel, umfassen;
h) Resten, die eine lodonium-, Sulfonium-, Oxonium, Ammonium-, Amidinium-, Guanidinium-, Pyridinium-, lmidazolium-, Triazolium-, Phosphonium- oder Carbonium-Gruppierung umfassen, wobei die Gruppierung vollständig oder teilweise die Rolle des Kations M übernimmt;
• -F, -Cl, -Br, -CN, -NO₂, -SCN und -N₃;
• -CₙF₂ₙ₊₁, -O-CₙF₂ₙ₊₁, -S-CₙF₂ₙ₊₁, -CH₂-CₙF₂ₙ₊₁, -OCF=CF₂ oder -SCF=CF₂, worin gilt: 1 ≤ n ≤ 8;
• Resten mit einer Wertigkeit v von zumindest 2, die v ionische Gruppen verbinden: 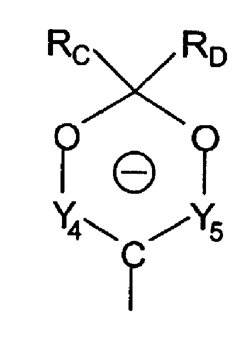
- alle Substituenten R_{C} und R_{D} jeweils unabhängig voneinander für einen einwertigen oder zweiwertigen organischen Rest stehen, der aus folgenden ausgewählt ist:
a) Alkyl, Alkenyl, Oxaalkyl, Oxaalkenyl, Azaalkyl, Azaalkenyl, Thiaalkyl, Thiaalkenyl, wobei die Reste gegebenenfalls eine Arylgruppe aufweisen;
b) Aryl, das gegebenenfalls zumindest einen Alkyl-, Alkenyl-, Oxa-alkyl-, Oxaalkenyl-, Azaalkyl-, Azaalkenyl-, Thiaalkyl- oder Thiaalkenylrest aufweist;
c) alizyklischen Resten oder aromatischen Resten, die gegebenenfalls zumindest eine Seitenkette, die ein Heteroatom aufweist, oder gegebenenfalls zumindest ein Heteroatom im Ring aufweisen;
d) oben unter a), b) und c) definierte Reste, die weiters Halogenatome aufweisen, in halogenierter oder perhalogenierter Form;
oder Teil einer Polymerkette sind, wobei zumindest einer der Substituenten ein perfluorierter Rest ist.

2. Verbindung nach Anspruch 1, **dadurch gekennzeichnet, dass** das organische Kation ein Oniumkation ist, das aus der aus Folgendem bestehenden Gruppe ausgewählt ist: den Kationen R₃O⁺ (Oxonium), NR₄⁺ (Ammonium), RC(NHR₂)₂⁺ (Amidinium), C(NHR₂)₃⁺ (Guanidinium), C₅R₆N⁺ (Pyridinium), C₃R₅N₂⁺ (lmidazolium), C₃R₇N₂⁺ (lmidazolinium), C₂R₄N₃⁺ (Triazolium), SR₃⁺ (Sulfonium), PR₄⁺ (Phosphonium), IR₂⁺ (lodonium), (C₆R₅)₃C⁺ (Carbonium), wobei die Reste R unabhängig voneinander H oder ein aus der aus Folgendem bestehenden Gruppe ausgewählter Rest sind:
- Alkyl-, Alkenyl-, Oxaalkyl-, Oxaalkenyl-, Azaalkyl-, Azaalkenyl-, Thiaalkyl-, Thiaalkenyl-, Silaalkyl-, Silaalkenyl-, Aryl-, Arylalkyl-, Alkylaryl-, Alkenylaryl-, Dialkylamino- und Dialkylazoresten;
- zyklischen oder heterozyklischen Resten, die gegebenenfalls zumindest eine Seitenkette umfassen, die Heteroatome, wie z.B. Stickstoff, Sauerstoff und Schwefel, umfasst;
- zyklischen oder heterozyklischen Resten, die gegebenenfalls Heteroatome im aromatischen Kern umfassen;
- Gruppen, die mehrere aromatische oder heterozyklische Kerne umfassen, die gegebenenfalls kondensiert sind und gegebenenfalls zumindest ein Stickstoff-, Sauerstoff-, Schwefel- oder Phosphoratom enthalten;
wobei es sich versteht, dass mehrere Reste R gemeinsam aliphatische oder aromatische Ringe bilden können, die gegebenenfalls ein Zentrum umschließen, das die kationische Ladung trägt.

3. Verbindung nach Anspruch 2, **dadurch gekennzeichnet, dass** das Oniumkation Teil des Rests Z ist.

4. Verbindung nach Anspruch 2, **dadurch gekennzeichnet, dass** das Oniumkation Teil einer Grundeinheit eines Polymers ist.

5. Verbindung nach Anspruch 2, **dadurch gekennzeichnet, dass** das Oniumkation eine kationische heterozyklische Gruppe mit aromatischem Charakter umfasst, die zumindest ein alkyliertes Stickstoffatom im Ring aufweist.

6. Verbindung nach Anspruch 5, **dadurch gekennzeichnet, dass** das Oniumkation eine Imidazolium-, Triazolium-, Pyridinium- oder 4-Dimethylaminopyridinium-Gruppe umfasst, wobei diese Gruppen gegebenenfalls einen Substituenten an den Ringkohlenstoffatomen aufweisen.

7. Verbindung nach Anspruch 2, **dadurch gekennzeichnet, dass** das Oniumkation eine Gruppierung, die einen Linker -N=N- oder -N=N⁺ aufweist, eine Sulfoniumgruppierung, eine lodoniumgruppierung oder ein gegebenenfalls substituiertes Aren-Ferrocenium-Kation ist und gegebenenfalls in ein Polymergerüst integriert ist.

8. Verbindung nach Anspruch 7, **dadurch gekennzeichnet, dass** das Oniumkation ein Diaryliodoniumkation, ein Dialkylaryliodoniumkation, ein Triarylsulfoniumkation, ein Trialkylarylsulfoniumkation oder ein gegebenenfalls substituiertes Phenacyldialkylsulfoniumkation ist.

9. Verbindung nach Anspruch 2, **dadurch gekennzeichnet, dass** das Oniumkation Teil eines Polymers ist.

10. Verbindung nach Anspruch 2, **dadurch gekennzeichnet, dass** das Oniumkation eine 2,2'-[Azobis(2,2'-imidazolinio-2-yl)propan]²⁺- oder eine 2,2'-Azobis(2-amidiniopropan)²⁺-Gruppe umfasst.

11. Verbindung nach Anspruch 1, **dadurch gekennzeichnet, dass** das Kation ein aus der aus Alkalimetallkationen, Erdalkalimetallkationen, Übergangsmetallkationen und Kationen dreiwertiger Metalle bestehenden Gruppe ausgewähltes Metallkation ist.

12. Verbindung nach Anspruch 1, **dadurch gekennzeichnet, dass** das Kation ein Metallocenium ist, das aus der aus Folgendem bestehenden Gruppe ausgewählt ist: von Ferrocen, Titanocen, Zirconocen abgeleiteten Kationen, Indenoceniumkationen, Arenmetalloceniumkationen, Kationen von mit Liganden vom Phosphintyp komplexierten Übergangsmetallen, die gegebenenfalls eine Chiralität aufweisen, und Organometall-Kationen, die eine oder mehrere Alkyl- oder Arylgruppen aufweisen, die kovalent an ein Atom oder eine Gruppe von Atomen gebunden sind, wobei die Kationen gegebenenfalls Teil einer Polymerkette sind.

13. Verbindung nach Anspruch 1, **dadurch gekennzeichnet, dass** einer der Substituenten R_{C} und R_{D} aus Alkylresten und Alkenylresten mit 1 bis 10 Kohlenstoffatomen ausgewählt ist.

14. Verbindung nach Anspruch 1, **dadurch gekennzeichnet, dass** einer der Substituenten R_{C} und R_{D} aus halogenierten oder perhalogenierten Alkyl- oder Alkenylresten mit 1 bis 10 Kohlenstoffatomen ausgewählt ist.

15. Verbindung nach Anspruch 1, **dadurch gekennzeichnet, dass** einer der Substituenten R_{C} und R_{D} aus Oxaalkyl- oder Oxaalkenylresten mit 1 bis 10 Kohlenstoffatomen ausgewählt ist.

16. Verbindung nach Anspruch 1, **dadurch gekennzeichnet, dass** Z ein R_{E}SO₂-Rest ist.

17. Verbindung nach Anspruch 1, **dadurch gekennzeichnet, dass** R_{E} oder R_{G} eine Gruppierung umfasst, die als Fänger freier Radikale geeignet ist und aus sterisch gehinderten Phenolen und Chinonen ausgewählt ist.

18. Verbindung nach Anspruch 1, **dadurch gekennzeichnet, dass** R_{E} oder R_{G} einen dissoziierenden Dipol umfasst, der aus Amid-, Sulfonamid- und Nitrilfunktionalitäten ausgewählt ist.

19. Verbindung nach Anspruch 1, **dadurch gekennzeichnet, dass** R_{E} oder R_{G} ein Redoxpaar umfasst, das aus einer Disulfidgruppe, einer Thioamidgruppe, einer Ferrocengruppe, einer Phenothiazingruppe, einer Bis(dialkylaminoaryl)gruppe, einer Nitroxidgruppe und einer aromatischen Imidgruppe ausgewählt ist.

20. Verbindung nach Anspruch 1, **dadurch gekennzeichnet, dass** R_{E}-Y_{E}- oder R_{E}R_{G}PO- optisch aktiv ist.

21. Verbindung nach Anspruch 1, **dadurch gekennzeichnet, dass** Z ein Teil einer Grundeinheit einer Polymerkette ist.

22. Verbindung nach Anspruch 1, **dadurch gekennzeichnet, dass** R_{E} oder R_{G} Teil eines Poly(oxyalkylen)rests oder eines Polystyrolrests ist.

23. Ionenleitfähiges Material, umfassend eine in einem Lösungsmittel gelöste ionische Verbindung, **dadurch gekennzeichnet, dass** die ionische Verbindung eine Verbindung nach Anspruch 1 ist.

24. Ionenleitfähiges Material nach Anspruch 23, **dadurch gekennzeichnet, dass** das Kation der ionischen Verbindung Ammonium, ein von einem Metall abgeleitetes Kation oder ein organisches substituiertes Ammonium-, Imidazolium-, Triazolium-, Pyridinium- oder 4-Dimethylaminopyridiniumkation ist, wobei die organischen Kationen gegebenenfalls einen Substituenten an den Ringkohlenstoffatomen aufweisen.

25. Ionenleitfähiges Material nach Anspruch 23, **dadurch gekennzeichnet, dass** die ionische Verbindung eine solche ist, dass zumindest einer der Substituenten R_{C}, R_{D}, R_{E} und R_{G} eine Alkyl-, Aryl-, Alkylaryl- oder Arylalkylgruppe oder eine Alkyl- oder Alkenylgruppe, die zumindest ein aus O, N und S ausgewähltes Heteroatom enthält, und/oder eine Hydroxy-, Carbonyl-, Amino- oder Carboxylgruppe ist.

26. Ionenleitfähiges Material nach Anspruch 23, **dadurch gekennzeichnet, dass** zumindest einer der Substituenten R_{E} und R_{G} aus Aryl-, Arylalkyl-, Alkylaryl- und Alkenylarylresten ausgewählt ist, worin die Seitenketten und/oder die aromatischen Kerne Heteroatome, wie z.B. Stickstoff, Sauerstoff oder Schwefel, umfassen.

27. Ionenleitfähiges Material nach Anspruch 23, **dadurch gekennzeichnet, dass** der Substituent Z aus der aus -OCₙF₂ₙ₊₁, -OC₂F₄H, -SCₙF₂ₙ₊₁, -SC₂F₄H, -OCF=CF₂, -SCF=CF₂ bestehenden Gruppe ausgewählt ist, worin n eine ganze Zahl von 1 bis 8 ist.

28. Ionenleitfähiges Material nach Anspruch 23, **dadurch gekennzeichnet, dass** der Substituent Z für eine Grundeinheit eines Polymers steht.

29. Ionenleitfähiges Material nach Anspruch 23, **dadurch gekennzeichnet, dass** das Lösungsmittel entweder ein flüssiges aprotisches Lösungsmittel, ausgewählt aus linearen Ethern, zyklischen Ethern, Estern, Nitrilen, nitrierten Derivaten, Amiden, Sulfonen, Sulfolanen, Sulfamiden und partiell halogenierten Kohlenwasserstoffen, ein polares Polymer oder ein Gemisch davon ist.

30. Ionenleitfähiges Material nach Anspruch 29, **dadurch gekennzeichnet, dass** das Lösungsmittel ein vernetztes oder unvernetztes solvatisierendes Polymer ist, das gegebenenfalls aufgepfropfte ionische Gruppen aufweist.

31. Ionenleitfähiges Material nach Anspruch 30, **dadurch gekennzeichnet, dass** das solvatisierende Polymer aus folgenden ausgewählt ist: aus gegebenenfalls ein Netzwerk bildenden Polyethern auf Poly(ethylenoxid)-Basis mit linearer, Kamm- oder Blockstruktur; Copolymeren, die ein Ethylenoxid- oder Propylenoxid- oder Allylglycidylether-Motiv enthalten; Polyphosphazenen; vernetzten Netzwerken auf Basis von mittels Isocyanaten vernetztem Polyethylenglykol; durch Polykondensation erhaltenen Netzwerken, die Gruppierungen aufweisen, die den Einbau von vernetzbaren Gruppierungen ermöglichen, und Blockcopolymeren, in denen bestimmte Blöcke Funktionalitäten mit Redoxeigenschaften aufweisen.

32. Ionenleitfähiges Material nach Anspruch 23, **dadurch gekennzeichnet, dass** das Lösungsmittel im Wesentlichen aus einem flüssigen aprotischen Lösungsmittel und einem polaren Polymerlösungsmittel besteht, das Einheiten umfasst, die zumindest ein aus Schwefel, Sauerstoff, Stickstoff und Fluor ausgewähltes Heteroatom enthalten.

33. Ionenleitfähiges Material nach Anspruch 32, **dadurch gekennzeichnet, dass** das polare Polymer vorwiegend von Acrylnitril, Vinylidenfluorid, N-Vinylpyrrolidon oder Methylmethacrylat abgeleitete Einheiten enthält.

34. Ionenleitfähiges Material nach Anspruch 23, **dadurch gekennzeichnet, dass** es weiters zumindest ein zweites Salz und/oder einen mineralischen oder organischen Füllstoff in Pulver- oder Faserform enthält.

35. Elektrochemischer Generator, umfassend eine negative Elektrode und eine positive Elektrode, die durch einen Elektrolyt getrennt sind, **dadurch gekennzeichnet, dass** der Elektrolyt ein Material nach einem der Ansprüche 23 bis 34 ist.

36. Generator nach Anspruch 35, **dadurch gekennzeichnet, dass** die negative Elektrode aus metallischem Lithium oder aus einer Legierung davon, gegebenenfalls in Form einer nanometrischen Dispersion in Lithiumoxid, oder aus einem Doppelnitrid von Lithium und einem Übergangsmetall oder aus einem Oxid der allgemeinen Formel Li_{1+y+x/3}Ti_{2-x/3}O₄ (0≤x≤1;0≤y≤1) oder aus Kohlenstoff und kohlenstoffhältigen Produkten, die durch Pyrolyse von organischen Materialien erhalten werden, besteht.

37. Generator nach Anspruch 35, **dadurch gekennzeichnet, dass** die positive Elektrode aus Vanadiumoxiden VOₓ (2 ≤ x ≤ 2,5), LiV₃O₈, Li_{y}Ni₁₋ₓCoₓO₂ (0 ≤ x ≤ 1; 0 ≤ y ≤ 1), Manganspinellen Li_{y}Mn₁₋ₓMₓO₂ (M = Cr, Al, V, Ni; 0 ≤ x ≤ 0,5; 0 ≤ y ≤ 2), organischen Polydisulfiden, FeS, FeS₂, Eisensulfat Fe₂(SO₄)₃, Eisen- und Lithiumphosphaten und -phosphosilicaten mit Olivinstruktur und den durch Substitution von Eisen durch Mangan erhaltenen Produkten, die einzeln oder in Form von Gemischen eingesetzt werden, besteht.

38. Generator nach Anspruch 35, **dadurch gekennzeichnet, dass** der Stromabnehmer der Kathode aus Aluminium besteht.

39. Superkondensator unter Verwendung zumindest einer Kohlenstoffelektrode mit hoher spezifischer Oberfläche oder zumindest einer ein Redoxpolymer enthaltenden Elektrode, worin der Elektrolyt ein Material nach einem der Ansprüche 23 bis 34 ist.

40. Verwendung eines Materials nach einem der Ansprüche 23 bis 34 zur p- oder n-Dotierung eines elektrisch leitfähigen Polymers.

41. Elektrochrome Vorrichtung, worin der Elektrolyt ein Material nach einem der Ansprüche 23 bis 34 ist.

42. Verfahren zur Polymerisation oder Vernetzung von Monomeren oder Präpolymeren, die kationisch reagieren können, **dadurch gekennzeichnet, dass** eine Verbindung nach Anspruch 1 als Photosäurebildner zum Katalysieren der Reaktion eingesetzt wird.

43. Verfahren nach Anspruch 42, **dadurch gekennzeichnet, dass** eine ionische Verbindung eingesetzt wird, deren Kation eine Gruppierung, die einen Linker -N=N⁺ oder -N=N- aufweist, eine Sulfoniumgruppe, eine Iodoniumgruppe oder ein substituiertes oder unsubstituiertes Aren-Ferrocen-Kation ist, wobei das Kation gegebenenfalls in ein Polymergerüst integriert ist.

44. Verfahren nach Anspruch 42, **dadurch gekennzeichnet, dass** die Monomere aus der aus Folgendem bestehenden Gruppe ausgewählt sind: Verbindungen, die eine zyklische Etherfunktionalität, eine zyklische Thioetherfunktionalität oder eine zyklische Aminfunktionalität umfassen, Vinylverbindungen, Vinylethern, Oxazolinen, Lactonen und Lactamen.

45. Verfahren nach Anspruch 42, **dadurch gekennzeichnet, dass** das Präpolymer aus der aus Verbindungen, in denen eine aliphatische Kette, eine aromatische Kette oder eine heterozyklische Kette Epoxygruppierungen aufweist, bestehenden Gruppe ausgewählt ist.

46. Verfahren nach Anspruch 42, **dadurch gekennzeichnet, dass** es darin besteht, den Photoinitiator mit zumindest einem Monomer oder Präpolymer zu vermischen, das kationisch polymerisiert werden kann, und das erhaltene Gemisch aktinischer Strahlung, einschließlich β-Strahlung, auszusetzen.

47. Verfahren nach Anspruch 42, **dadurch gekennzeichnet, dass** das Reaktionsgemisch Strahlung ausgesetzt wird, nachdem es in die Form einer dünnen Schicht gebracht wurde.

48. Verfahren nach Anspruch 42, **dadurch gekennzeichnet, dass** der Photoinitiator in Form einer Lösung in einem gegenüber der Polymerisationsreaktion inerten Lösungsmittel eingesetzt wird.

49. Verfahren nach Anspruch 48, **dadurch gekennzeichnet, dass** das inerte Lösungsmittel aus der aus Aceton, Methylethylketon, Acetonitril, Propylencarbonat, γ-Butyrolacton, Etherestern von Mono-, Di-, Triethylen- oder -propylenglykolen, Etheralkoholen von Mono-, Di-, Triethylen- oder -propylenglykolen sowie Phthalsäure- oder Zitronensäureestern bestehenden Gruppe ausgewählt ist.

50. Verfahren nach Anspruch 42, **dadurch gekennzeichnet, dass** die Reaktion in Gegenwart eines Lösungsmittels oder eines Verdünnungsmittels erfolgt, das aus einer in Bezug auf die Polymerisation reaktiven Verbindung besteht.

51. Verfahren nach Anspruch 42, **dadurch gekennzeichnet, dass** die reaktive Verbindung aus Vinylmono- und -diethern von Mono-, Di-, Tri-, Tetraethylen- oder -propylenglykolen, Trivinylethertrimethylolpropan und Dimethanolcyclohexandivinylether, N-Vinylpyrrolidon und Propylencarbonat-2-propenylether ausgewählt ist.

52. Verfahren nach Anspruch 42, **dadurch gekennzeichnet, dass** ein Photosensibilisator zu dem Reaktionsgemisch zugesetzt wird.

53. Verfahren nach Anspruch 52, **dadurch gekennzeichnet, dass** der Photosensibilisator aus der aus Folgendem bestehenden Gruppe ausgewählt ist: Anthracen, 9,10-Diphenylanthracen, Perylen, Phenothiazin, Tetracen, Xanthon, Thioxanthon, Isopropylthioxanthon, Acetophenon, Benzophenon, 1,3,5-Triaryl-2-pyrazolinen und Derivaten davon, die durch Substitution der aromatischen Kerne mit Alkyl-, Oxa- oder Azaalkylresten erhalten werden.

54. Verfahren nach Anspruch 42, **dadurch gekennzeichnet, dass** das Reaktionsgemisch weiters zumindest ein Monomer oder Präpolymer, das radikalisch polymerisiert werden kann, oder eine Verbindung enthält, die unter Einwirkung der aktinischen Strahlung oder β-Strahlung oder unter Einwirkung von Wärme einen radikalischen Polymerisationsinitiator freisetzen kann.

55. Verfahren zur Modifikation der Löslichkeitseigenschaften eines Polymers, das säureempfindliche Gruppen aufweist, **dadurch gekennzeichnet, dass** das Verfahren darin besteht, das Polymer in Gegenwart einer Verbindung nach Anspruch 1 aktinischer Strahlung oder β-Strahlung auszusetzen.

56. Verfahren nach Anspruch 55, **dadurch gekennzeichnet, dass** das Polymer von tertiären Alkoholen abgeleitete Ester- oder Arylethermotive enthält.

57. Verfahren nach Anspruch 56, **dadurch gekennzeichnet, dass** das Polymer aus der aus tert-Butylpolyacrylaten, tert-Butyl- oder tert-Amylpolyitaconaten, Poly(tert-butoxycarbonyloxystyrol) und Poly(tert-butoxystyrol) bestehenden Gruppe ausgewählt ist.

58. Verfahren nach Anspruch 55, **dadurch gekennzeichnet, dass** es durchgeführt wird, um Photoresists chemisch zu verstärken.

59. Kationische Farbstoffzusammensetzung, **dadurch gekennzeichnet, dass** sie eine Verbindung nach Anspruch 1 enthält.

60. Kationische Farbstoffzusammensetzung nach Anspruch 59, **dadurch gekennzeichnet, dass** die negative(n) Ladung(en) der anionischen Gruppe entweder an das Farbstoffmolekül gebunden ist/sind oder ein Gegenion für die positiven Ladungen des Farbstoffs darstellt/darstellen.

61. Verwendung einer Verbindung nach Anspruch 1 als Katalysator in Friedel-Crafts-Reaktionen, Diels-Alder-Reaktionen, Aldolisationsreaktionen, Michael-Additionen, Allylierungsreaktionen, Pinakol-Kupplung, Glykosylierungsreaktionen, Oxetan-Ringöffnungsreaktionen, Metathesereaktionen von Alkenen, Ziegler-Natta-Polymerisationen, Metathesepolymerisationen unter Ringöffnung und Metathesepolymerisationen von azyklischen Dienen.

62. Verwendung nach Anspruch 61, **dadurch gekennzeichnet, dass** das Kation der Verbindung aus Lithium, Magnesium, Kupfer, Zink, Zinn, dreiwertigen Metallen, einschließlich Seltenerdmetallen, Platinoiden und Organometall-Kationen ausgewählt ist.

63. Verwendung einer Verbindung nach Anspruch 6 als Lösungsmittel zur Durchführung von chemischen, photochemischen, elektrochemischen und photoelektrochemischen Reaktionen, wobei die Verbindung bei Temperaturen oberhalb ihres Schmelzpunkts eingesetzt wird.

64. Elektrisch leitfähiges Material, **dadurch gekennzeichnet, dass** es eine Verbindung nach Anspruch 1 enthält.

65. Elektrisch leitfähiges Material nach Anspruch 64, **dadurch gekennzeichnet, dass** zumindest einer der Substituenten des anionischen aromatischen Heterozyklus in der ionischen Verbindung eine Alkylkette mit 6 bis 20 Kohlenstoffatomen enthält.

66. Elektrisch leitfähiges Material nach Anspruch 64, **dadurch gekennzeichnet, dass** der kationische Anteil der ionischen Verbindung ein Polykation ist, das aus einem konjugierten, "p"-dotierten Polymer besteht.
